# EUROPEAN PATENT SPECIFICATION

(11) **EP 2 377 907 B1**
(45) Date of publication and mention of the grant of the patent: **03.12.2014**
(21) Application number: 09836312.0
(22) Date of filing: 16.12.2009
(51) Int. Cl.: C09K 11/06, C07D 263/56, C07D 277/66, H01L 51/00, H05B 33/10, H01L 51/50

(54) **NOVEL COMPOUNDS FOR AN ORGANIC PHOTOELECTRIC DEVICE, AND ORGANIC PHOTOELECTRIC DEVICE COMPRISING SAME**
NEUE VERBINDUNGEN FÜR ORGANISCHE PHOTOELEKTRISCHE ANORDNUNG SOWIE SIE ENTHALTENDE ORGANISCHE PHOTOELEKTRISCHE ANORDNUNG
NOUVEAUX COMPOSÉS POUR DISPOSITIF PHOTOÉLECTRIQUE ORGANIQUE ET DISPOSITIF PHOTOÉLECTRIQUE ORGANIQUE LES COMPRENANT

(30) Priority: 30.12.2008 KR 20080137222
(43) Date of publication of application: 19.10.2011
(73) Proprietor: CHEIL INDUSTRIES INC., Kumi-city, Kyungsangbuk-do 730-030 (KR)
(72) Inventor: IN, Kyu-Yeol, Uiwang-si Gyeonggi-do 437-711 (KR); KANG, Myeong-Soon, Uiwang-si Gyeonggi-do 437-711 (KR); JUNG, Ho-Kuk, Uiwang-si Gyeonggi-do 437-711 (KR); KIM, Nam-Soo, Uiwang-si Gyeonggi-do 437-801 (KR); KANG, Eui-Su, Uiwang-si Gyeonggi-do 437-711 (KR); CHAE, Mi-Young, Uiwang-si Gyeonggi-do 437-711 (KR); PARK, Jin-Seong, Uiwang-si Gyeonggi-do 437-801 (KR)
(74) Representative: Michalski Hüttermann & Partner Patentanwälte mbB
(86) International application number: PCT/KR2009/007521
(87) International publication number: WO 2010/076991

(56) References cited:
- EP-A1- 1 734 038
- WO-A1-2008/082249
- WO-A2-03/060956
- WO-A2-2009/051454
- JP-A- 2002 038 141
- JP-A- 2002 212 181
- JP-A- 2002 212 181
- JP-A- 2003 268 362
- JP-A- 2005 044 790
- JP-A- 2006 265 515
- JP-A- 2006 265 515
- KR-A- 20060 023 046
- US-A1- 2007 069 638
- US-A1- 2007 205 412
- CHEN ET AL: "Synthesis and characterization of cyclometalated iridium(III) complexes containing benzoxazole derivatives and different ancillary ligands", JOURNAL OF ORGANOMETALLIC CHEMISTRY, ELSEVIER-SEQUOIA S.A. LAUSANNE, CH, vol. 693, no. 19, 15 September 2008 (2008-09-15), pages 3117-3130, XP024528185, ISSN: 0022-328X, DOI: 10.1016/J.JORGANCHEM.2008.06.034 [retrieved on 2008-07-04]

## Description

### [Technical Field]

The present invention relates to a novel compound for an organic photoelectric device and an organic photoelectric device including the same. More particularly, the present invention relates to a material for an organic photoelectric device that decreases a driving voltage of an organic photoelectric device due to excellent thermal stability and improves life-span and efficiency and an organic photoelectric device including the same.

### [Background Art]

An organic photoelectric device transforms electrical energy into light by applying current to an organic light emitting material. It has a structure in which a functional organic material layer is interposed between an anode and a cathode.

The organic light emitting diode has similar electrical characteristics to those of light emitting diodes (LEDs) in which holes are injected from an anode and electrons are injected from a cathode, then the holes and electrons move to opposite electrodes and are recombined to form excitons having high energy. The formed excitons generate light having a certain wavelength while shifting to a ground state.

Light emission of an anthracene organic material was firstly found in 1960, but there is a demerit of a high driving voltage.

In JP 2006 265515 A a light-emitting device material is described, for example compound 21, containing a pyrene compound represented by the following general formula (1): wherein R1 to R10 are specific functional groups, provided that at least one of the R1 to R10 is a substituent represented by the following general formula (2): wherein R11 to R14 are specific functional groups, provided that any one of the R11 to R14 is used for a single bond with a pyrene skeleton; X1 is a group represented by the following general formula (3): wherein R15 is a specific functional group; and Y1 to Y4 is selected from among a nitrogen atom and a carbon atom, provided that at least one of the Y1 to Y4 is a nitrogen atom and at least one thereof is a carbon atom, and no substituents exist on the nitrogen atom in the case of the nitrogen atom. This material provides a light-emitting device having high luminous efficiency and excellent durability.

WO 03/060956 A2 describes novel materials for electron injection/transportation and emitting layers can greatly improve the stability of an organic electroluminescent display. Electroluminescent displays incorporating these materials produce blue light at low voltage levels. These novel organic materials include compounds, for example 2 and 3, in which 1 to 2 imidazole functional groups are introduced in the 2 or 2,6-site of 9,10 substituted anthracene. An organic electroluminescent display with an organic compound layer of these materials has high efficiency, thermal stability, operationally stability and maintains driving voltage before and after operation.

JP 2005044790 A describes organic electroluminescent element having at least a light emitting layer containing a phosphorescent compoundbetween an anode and a cathode having an adjacent layer disposed between the light emitting layer and the cathode and adjacent to the light emitting layer, and including a compound, for example compound 14, represented by a general formula (1) in the adjacent layer.

US 2007205412 A1 describes a novel anthracene derivative and an organic electronic device using the same. The organic electronic device shows excellent characteristics in efficiency, drive voltage, and life time.

WO 2008/082249 A1 describes organic electroluminescent compounds and electroluminescent devices comprising the same as host material. The electroluminescent compounds having three ligands, two bivalent metals and a monovalent anion derived from an inorganic or an organic acid.

JP 2002212181 A describes a method for producing the benzazole group-containing triazine compound by trimerizing a compound.

Cyclometalated iridium (III) complexes containing benzoxazole derivatives and different ancillary ligands are described in CHEN ET AL: "Synthesis and characterization of cyclometalated iridium(III) complexes containing benzoxazole derivatives and different ancillary ligands", JOURNAL OF ORGANOMETALLIC CHEMISTRY, ELSEVIER-SEQUOIA S.A. LAUSANNE, CH, vol. 693, no. 19, 15 September 2008 (2008-09-15), pages 3117-3130, XP024528185, ISSN: 0022-328X, DOI: 10.1016/J.JORGANCHEM.2008.06.034 [retrieved on 2008-07-04].

In US 2007/069638 A1 a blue organic electroluminescent device (1) is described including at least an emitting layer (40) between an anode (20) and a cathode (60), the emitting layer (40) including a host material and a plurality of dopants, at least one of the dopants having ultraviolet luminescent properties.

WO 2009/051454 A2 describes a compound for an organic photoelectric device and an organic photoelectric device including the same, wherein the compound can act as a hole injection, hole transport, light emitting, or electron injection and/or transport material.

In EP 1 734 038 A1 a material for an organic EL device is described realizing an organic EL device capable of having a high current efficiency even at a low voltage. EP 1 734 038 A1 described a derivative of heterocyclic compound, for example compounds 2 to 25, having a nitrogen atom represented by the following general formula (A-1) or (A-2). In the formulae, R 1a to R 5a each represent a substituent, Ar 1a to Ar 3a each represent a single bond or a divalent connecting group, and HAr represents a group represented by a general formula (A-3) or (A-4). R 6a to R 10a each represent a substituent.

In 1980, C.W Tang et al., of Eastman Kodak, Inc., firstly developed a device realizing a low driving voltage. Thereafter, in 1990, Cambridge University developed a polymer light emitting diode using poly(p-phenylenevinylene) (PPV). Research has been conducted on a low molecular light emitting element (SMOLED) and a polymer organic light emitting diode (PLED). The low molecular organic light emitting diode is manufactured as a thin film in a vacuum deposition method, and can have good efficiency and life-span performance. A polymer organic light emitting diode is manufactured in an Inkjet or spin coating method and has an advantage of low initial cost and being large-sized.

Both low molecular organic light emitting and polymer organic light emitting diodes have advantages of being self-light emitting and ultrathin, and having a high speed response, a wide viewing angle, high image quality, durability, a large driving temperature range, and the like. In particular, they have good visibility due to the self-light emitting characteristic compared with a conventional LCD (liquid crystal display) and have an advantage of decreasing thickness and weight of LCD by up to a third, because they do not need a backlight. In addition, since they have a response speed that is 1000 times faster per microsecond unit than an LCD, they can realize a perfect motion picture without an after-image. Based on these advantages, they have been remarkably developed to have 80 times the efficiency and more than 100 times the life-span since they were first developed in the late 1980s. Recently, these diodes have been used in displays that are rapidly becoming larger, such as for a 40-inch organic light emitting diode panel.

These displays must simultaneously have improved luminous efficiency and life-span in order to be larger. In order to increase the luminous efficiency, smooth combination between holes and electrons in an emission layer is needed. However, since an organic material in general has slower electron mobility than hole mobility, it has a drawback of inefficient combination between holes and electrons. Accordingly, a novel compound that increases electron injection and mobility from a cathode and simultaneously prevents movement of holes is desperately required.

In addition, the device may have a decreased life-span since the material therein may be crystallized due to Joule heat generated when it is driven. In order to solve this problem, 2-(4-biphenylyl)-5- (4-t-butylphenyl)-1,3,4-oxadiazole (PBD) (Jpn. J. Appl. Phys., 27, L269 1988) with a rapid transfer speed has been suggested, but it lacks thermal stability and thereby still has a problem of crystallization when a device is driven. In addition, BCP and an aluminum mixed coordination complex (BAlq, bis(2-methyl-8-quinolinolate)-4-(phenylphenolate)aluminum), which is known to be excellent in lowering hole mobility, have been actively researched. However, these materials have excellent characteristic in lowering hole mobility but a problem of deteriorating the electron injection characteristic and being crystallized when a device is driven, and accordingly do not satisfy device characteristics.

Therefore, there is a strong need for an organic compound having excellent electron injection and mobility and high thermal stability and that prevents crystallization when a device is driven.

### [DISCLOSURE]

### [Technical Problem]

The subject matter of the present invention is represented by the claims 1 to 12.

An exemplary embodiment of the present invention provides a novel material that can act as a hole injection, hole transport, light emitting, or electron injection and/or transport material, and also as a light emitting host along with an appropriate dopant.

Another embodiment of the present invention provides an organic photoelectric device including the material for an organic photoelectric device, and having decreased driving voltage and increased life-span and efficiency.

### [Technical Solution]

According to one embodiment of the present invention, a compound for an organic photoelectric device, represented by the following Chemical Formula 112: 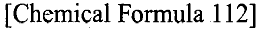 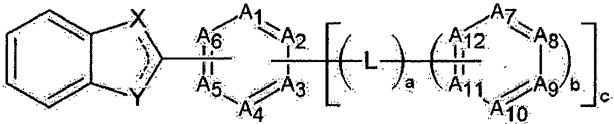 wherein, in Chemical Formula 112,
A₁ to A₁₂ are the same or different, and independently selected from the group consisting of CR₁ to CR₁₂ and N, provided that at least one of A₇ to A₁₂ is N, and R₁ to R₆ adjacent to each other optionally forms a fused ring, and R₇ to R₁₂ adjacent to each other optionally forms a fused ring,
X is selected from the group consisting of O, S, Se and NR₁₃, Y is CR₁₄ or N, provided that when X is selected from the group consisting of O, S, and Se, and Y is N,
L is a substituted or unsubstituted C6 to C50 arylene,
a is 0 or 1, provided that when a is 0, A₁ to A₆ are each independently CR₁ to CR₆, and when a is 1, at least one of A₁ to A₆ is N,
b and c are the same or different, and independently an integer ranging from 1 to 3, and
R₁ to R₁₄ are the same or different, and independently selected from the group consisting of hydrogen, a halogen, a nitrile, a cyano, a nitro, an amide, a carbonyl, an ester, a substituted or unsubstituted alkyl, a substituted or unsubstituted alkylene, a substituted or unsubstituted alkoxy, a substituted or unsubstituted alkenyl, a substituted or unsubstituted alkenylene, a substituted or unsubstituted alkynyl, a substituted or unsubstituted alkynylene, a substituted or unsubstituted cycloalkyl, a substituted or unsubstituted cycloalkylene, a substituted or unsubstituted aryl, a substituted or unsubstituted arylene, a substituted or unsubstituted arylamine, a substituted or unsubstituted heteroarylamine, a substituted or unsubstituted heterocycle, a substituted or unsubstituted amino, BRR', or SiRR'R", wehrein R, R' and R"are the same or different, and each independently a substituted or unsubstituted alkyl, or a substituted or unsubstituted aryl.

According to another embodiment of the present invention, acompound for an organic photoelectric device, represented by the following Chemical Formula 113: 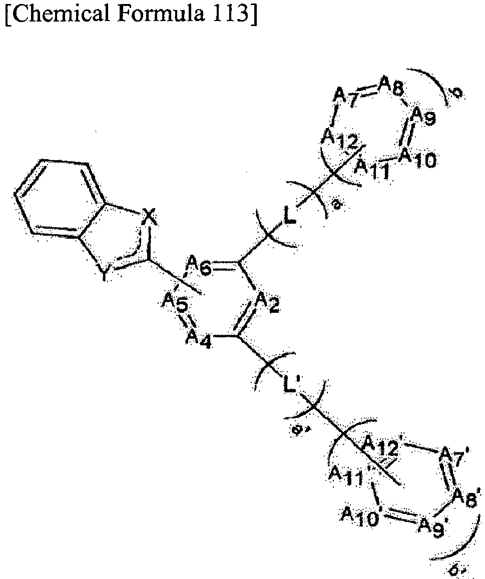 wherein, in Chemical Formula 113,
A₂, A₄ to A₁₂ and A₇' to A₁₂' are the same or different, and are each independently selected from the group consisting of CR₂, CR₄ to CR₁₂, CR₇' to CR₁₂' and N, provided at least one of A₇ to A₁₂ is N, and at least one of A₇' to A₁₂' is N, wherein R₄ to R₆ adjacent to each other optionally forms a fused ring, R₇ to R₁₂ adjacent to each other optionally forms a fused ring, and R₇' to R₁₂' adjacent to each other optionally forms a fused ring,
X is selected from the group consisting of O, S, Se and NR₁₃, Y is CR₁₄ or N, provided that when X is selected from the group consisting of O, S, and Se, and Y is N,
L and L' are the same or different, and are each independently is a substituted or unsubstituted C6 to C50 arylene,
a and a' are the same or different, and are each independently 0 or 1, provided that when a and a' are each independently 0, A₂, A₄ to A₆ are each independently CR₂, and CR₄ to CR₆, and when a and a' are each independently 1, at least one of A₂, and A₄ to A₆ is N,
b and b' are the same or different, and are each independently an integer ranging from 1 to 3, and
R₂, R₄ to R₁₄, and R₇' to R₁₂' are the same or different, and independently hydrogen, a halogen, a nitrile, a cyano, a nitro, an amide, a carbonyl, an ester, a substituted or unsubstituted alkyl, a substituted or unsubstituted alkylene, a substituted or unsubstituted alkoxy, a substituted or unsubstituted alkenyl, a substituted or unsubstituted alkenylene, a substituted or unsubstituted alkynyl, a substituted or unsubstituted alkynylene, a substituted or unsubstituted cycloalkyl, a substituted or unsubstituted cycloalkylene, a substituted or unsubstituted aryl, a substituted or unsubstituted arylene, a substituted or unsubstituted arylamine, a substituted or unsubstituted hetero arylamine, a substituted or unsubstituted heterocycle, a substituted or unsubstituted amino, BRR', or SiRR'R", wehrein R, R' and R"are the same or different, and each independently a substituted or unsubstituted alkyl, or a substituted or unsubstituted aryl.

According to another embodiment of the present invention, an organic photoelectric device of the present invention is provided that includes an anode, a cathode, and at least one organic thin layer between the anode and cathode. The organic thin layer includes the compound for an organic photoelectric device.

According to a further of the present invention, a display device including the organic photoelectric device is provided.

Hereinafter, further embodiments of the present invention will be described in detail.

### [Advantageous Effects]

According to one embodiment of the present invention, a compound for an organic photoelectric device is largely asymmetric. The asymmetric structure reinforces an amorphous characteristic and thereby suppresses crystallization, improving thermal stability of the compound for an organic photoelectric device. Accordingly, when an organic photoelectric device is driven, the compound can decrease its driving voltage and improve life-span and efficiency.

The novel compound according to one embodiment of the present invention can act as a hole injection, hole transport, light emitting, or electron inject ion and/or transport material, and also as a light emitting host along with an appropriate dopant.

### [Description of the Drawings]

FIGS. 1 to 5 are cross-sectional views showing organic photoelectric devices including organic compounds according to various embodiments of the present invention.

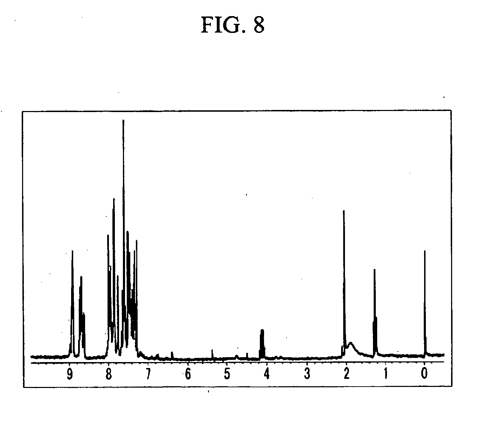

FIG. 8 shows a ¹H NMR spectrum of the compound according to Example 3.

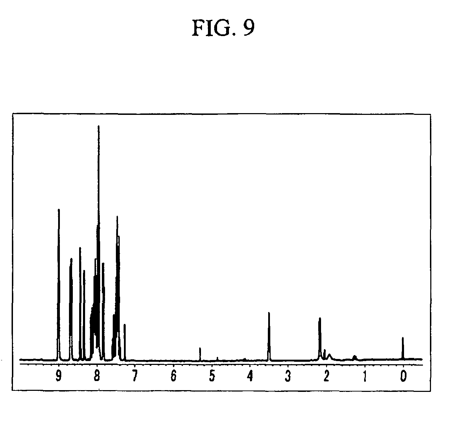

FIG. 9 shows a ¹H NMR spectrum of the compound according to Example 4.

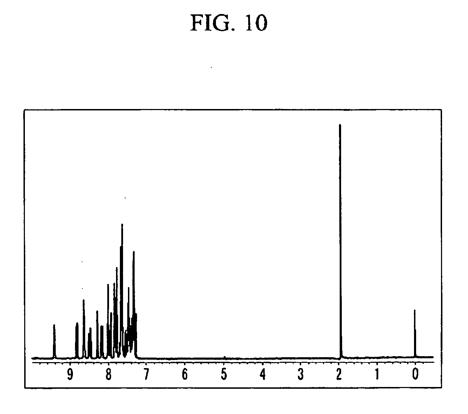

FIG. 10 shows a ¹H NMR spectrum of the compound according to Example 5.

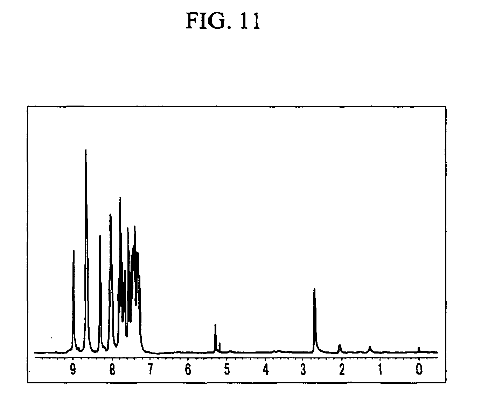

FIG. 11 shows a ¹H NMR spectrum of the compound according to Example 6.

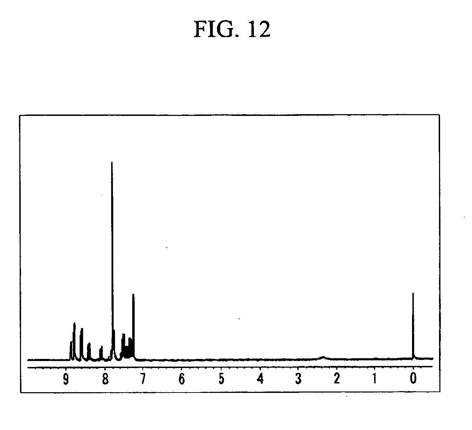

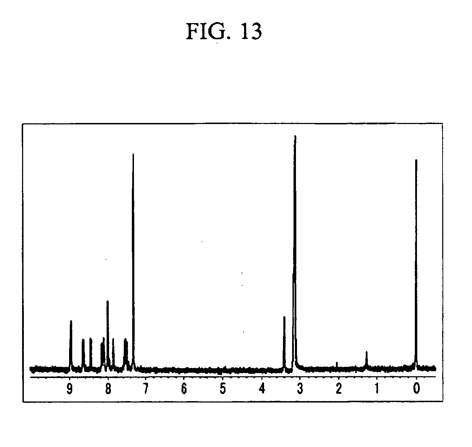

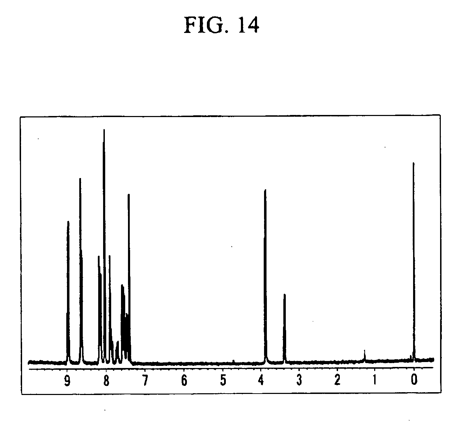

FIG. 12 shows a ¹H NMR spectrum of the compound according to Example 7.
FIG. 15 shows voltage-luminescence characteristics of the organic light emitting diodes according to Examples 11 to 13 of the invention and Comparative Example 2.
FIG. 16 shows voltage-luminescence characteristics of the organic light emitting diodes according to Example 14 of the invention and Comparative Example 2.
FIG. 17 shows electrical power efficiency of the organic light emitting diodes according to Examples 11 to 13 of the invention and Comparative Example 2.
FIG. 18 shows electrical power efficiency of the organic light emitting diodes according to Example 14 of the invention and Comparative Example 2.
FIG. 19 shows thermal properties of the compound according to Example 5.
FIG. 20 shows thermal properties of the compound according to Comparative Example 1.

### <Description of Reference Numerals Indicating Primary Elements in the Drawings>

100 : organic photoelectric device 110: cathode
120 : anode 105 : organic thin layer
130 : emission layer 140 : hole transport layer (HTL)
150 : electron transport layer (ETL) 160 : electron injection layer (EIL)
170 : hole injection layer (HIL) 230 : emission layer + electron transport layer (ETL)

### [Mode for Invention]

Exemplary embodiments of the present invention will hereinafter be described in detail. However, these embodiments are only exemplary, and the present invention is not limited thereto but rather is defined by scope of the appended claims.

As used herein, when specific definition is not provided, the term "alkyl" may refer to a C1 to C30 alkyl, and preferably a C1 to C15 alkyl; the term "alkoxy" may refer to a C1 to 30 alkoxy, and preferably a C1 to C15 alkoxy; the term "alkenyl" may refer to a C 2 to C30 alkenyl, and preferably a C2 to C15 alkenyl; the term "cycloalkyl" may refer to a C 3 to C30 cycloalkyl, and preferably a C3 to C15 cycloalkyl; the term "aryl" may refer to a C 6 to C50 aryl, and preferably a C6 to C25 aryl; the term "arylamine" may refer to a C 7 to C50 arylamine, and preferably a C7 to C25 arylamine; the term "heteroarylamine" may refer to a C7 to C50 heteroarylamine, and preferably a C7 to C25 hetero arylamine; the term "heterocycle" may refer to a C5 to C50 heterocycle, and preferably a C5 to C25 heterocycle; and the term "fluoroalkyl" may refer to a C 1 to C 10 fluoroalkyl.

As used herein, when a definition is not otherwise provided, the term "ester" may refer to-COOR; and the term "carbonyl" may refer to -COR'. Herein R and R'are each independently hydrogen, a C1 to C10 alkyl, a C 6 to C20 aryl, a C3 to C20 cycloalkyl, a C2 to C10 alkenyl, a C2 to C10 alkynyl, or a combination thereof.

As used herein, when specific definition is not otherwise provided, the term "substituted" refers to one substituted with at least a substituent selected from the group consisting of a halogen, a nitrile, a cyano, a nitro, an amide, a carbonyl, an acetylene, a substituted or unsubstituted alkyl, a substituted or unsubstituted alkenyl, a substituted or unsubstituted alkoxy, a substituted or unsubstituted arylamine, a substituted or unsubstituted aryl, a substituted or unsubstituted arylalkyl, a substituted or unsubstituted arylalkenyl, a substituted or unsubstituted heterocycle, a substituted or unsubstituted amine, a substituted or unsubstituted cycloalkyl, BR₆R₇, and SiR₆R₇R₈, where R₆ to R₈ are the same or different and are independently selected from the group consisting of a substituted or unsubstituted C1 to C30 alkyl and a substituted or unsubstituted C6 to C50 aryl.

As used herein, when a definition is not otherwise provided, the prefix "hetero" refers to one including 1 to 3, including N, O, S, or P, in one ring.

As used herein, when specific definition is not provided, the term "heterocycle" refers to one selected from the group consisting of a C5 to C50 heteroaryl, a C5 to C50 heterocycloalkyl, a C5 to C50 heterocycloalkenyl, a C5 to C50 heterocycloalkynyl, and fused rings thereof The heterocycle preferably includes 1 to 20 heteroatoms, and more preferably 1 to 15 heteroatoms.

In one embodiment of the present invention, the compound for an organic photoelectric device represented by the following Chemical Formula 112 includes sequentially repeated aryls and heterocycles.

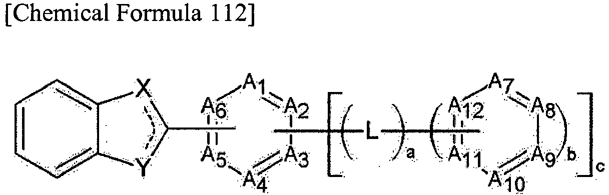

In Chemical Formula 112, A₁ to A₁₂ are the same or different, and independently selected from the group consisting of CR₁ to CR₁₂ and N, provided that at least one of A₇ to A₁₂ is N, and R₁ to R₆ adjacent to each other can form a fused ring, and R₇ to R₁₂ adjacent to each other can form a fused ring.

X is selected from the group consisting of O, S, Se and NR₁₃, Y is CR₁₄ or N, provided that when X is selected from the group consisting of O, S, and Se, and Y is N,
L is a substituted or unsubstituted C6 to C50 arylene,
a is 0 or 1, provided that when a is 0, A₁ to A₆ are each independently CR₁ to CR₆, and when a is 1, at least one of A₁ to A₆ is N,
b and c are the same or different, and independently an integer ranging from 1 to 3, and
R₁ to R₁₄ are the same or different, and independently selected from the group consisting of hydrogen, a halogen, a nitrile, a cyano, a nitro, an amide, a carbonyl, an ester, a substituted or unsubstituted alkyl, a substituted or unsubstituted alkylene, a substituted or unsubstituted alkoxy, a substituted or unsubstituted alkenyl, a substituted or unsubstituted alkenylene, a substituted or unsubstituted alkynyl, a substituted or unsubstituted alkynylene, a substituted or unsubstituted cycloalkyl, a substituted or unsubstituted cycloalkylene, a substituted or unsubstituted aryl, a substituted or unsubstituted arylene, a substituted or unsubstituted arylamine, a substituted or unsubstituted heteroarylamine, a substituted or unsubstituted heterocycle, a substituted or unsubstituted amino, BRR', or SiRR'R", wehrein R, R' and R"are the same or different, and each independently a substituted or unsubstituted alkyl, or a substituted or unsubstituted aryl.

When at least adjacent two of R₁ to R₆ form a fused ring, or at least adjacent two of R₇ to R₁₂ form a fused ring, a fused ring including A₁ to A₆ and a fused ring including A₇ to A₁₂ may be polycyclic aryls such as naphthyl, anthracenyl, phenanthrenyl, pyrenyl, peryenyl, pyrenyl, fluorenyl, and the like. Particularly, when at least one of A₇ to A₁₂ is N, one or more carbons of the polycyclic aryls may be substituted with N.

When R₇ to R₁₂ are each independently substituted or unsubstituted aryl or substituted or unsubstituted arylene, the compound may be usefully applicable to an emission layer.

When X is selected from the group consisting of O, S and Se, Y is N. That is to say, at least one of X and Y is essentially N. In this case, it can lower the LUMO (lowest unoccupied molecular orbital) energy level and increase electron affinity of a molecule, and thereby improve injection and transport characteristics of electrons. Accordingly, it can reduce voltage required for driving an organic light emitting diode and improve electrical power efficiency.

The functional substitutents including X and Y provide the compound with a predetermined rigidity and thus increase intermolecular interaction. The compound for an organic photoelectric device according to one embodiment has thermal stability due to a high glass transition temperature, and thereby improves life-span of an organic light emitting diode.

L is a substituted or unsubstituted C6 to C50 arylene. More particularly, aryls of R₇ to R₁₂ and L is a C6 to C50 aryl, and the aryl may be monocyclic aryls such as phenyl; or or polycyclic aryls such as naphthyl, anthracenyl, phenanthrenyl, pyrenyl, peryenyl, and so on. The compound may be useful for a material of an emission layer.

The linking group, L can increase intermolcular interaction, and thereby improve thermal stability. In addition, it can adjust the π-conjugation length and also light emitting in a visual region. Accordingly, a compound for an organic photoelectric device according to the present invention can be usefully applied to an emission layer.

b and c are integers ranging from 1 to 3, and when b and c are 2 or more, each repeating unit may be different.

Particularly, when c is 2, two repeating units are linked to each other at a meta position of the benzene ring including A₁ to A₆. The compound for an organic photoelectric device according to one embodiment may include the compound represented by the following Chemical Formula 113:

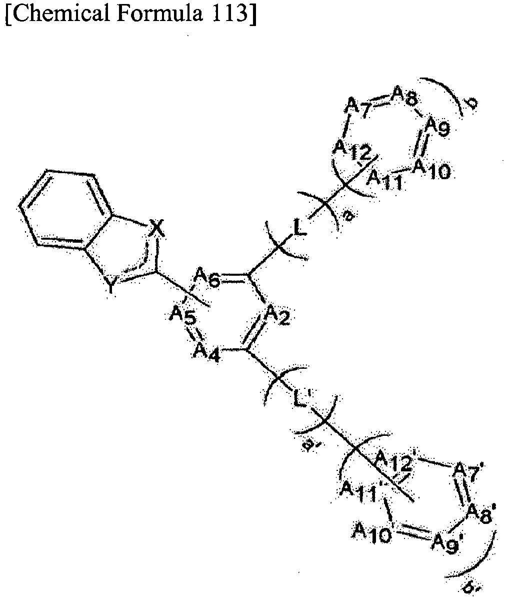

In Chemical Formula 113,
A₂, A₄ to A₁₂ and A₇' to A₁₂' are the same or different, and are each independently selected from the group consisting of CR₂, CR₄ to CR₁₂, CR₇' to CR₁₂' and N, provided at least one of A₇ to A₁₂ is N, and at least one of A₇' to A₁₂' is N, wherein R₄ to R₆ adjacent to each other can form a fused ring, R₇ to R₁₂ adjacent to each other can form a fused ring, and R₇' to R₁₂' adjacent to each other can form a fused ring,
X is selected from the group consisting of O, S, Se and NR₁₃, Y is CR₁₄ or N, provided that when X is selected from the group consisting of O, S, and Se, and Y is N,
L and L' are the same or different, and are each independently is a substituted or unsubstituted C6 to C50 arylene,
a and a' are the same or different, and are each independently 0 or 1, provided that when a and a' are each independently 0, A₂, A₄ to A₆ are each independently CR₂, and CR₄ to CR₆, and when a and a' are each independently 1, at least one of A₂, and A₄ to A₆ is N,
b and b' are the same or different, and independently an integer ranging from 1 to 3, and
R₂, R₄ to R₁₄, and R₇' to R₁₂' are the same or different, and independently hydrogen, a halogen, a nitrile, a cyano, a nitro, an amide, a carbonyl, an ester, a substituted or unsubstituted alkyl, a substituted or unsubstituted alkylene, a substituted or unsubstituted alkoxy, a substituted or unsubstituted alkenyl, a substituted or unsubstituted alkenylene, a substituted or unsubstituted alkynyl, a substituted or unsubstituted alkynylene, a substituted or unsubstituted cycloalkyl, a substituted or unsubstituted cycloalkylene, a substituted or unsubstituted aryl, a substituted or unsubstituted arylene, a substituted or unsubstituted arylamine, a substituted or unsubstituted hetero arylamine, a substituted or unsubstituted heterocycle, a substituted or unsubstituted amino, BRR', or SiRR'R", wehrein R, R' and R"are the same or different, and each independently a substituted or unsubstituted alkyl, or a substituted or unsubstituted aryl.

At least adjacent two of R₄ to R₆ can form a fused ring, at least adjacent two of R₇ to R₁₂ can form a fused ring, and at least adjacent two of R₇' to R₁₂' can form a fused ring. The fused ring may be polycyclic aryls such as naphthyl, anthracenyl, phenanthrenyl, pyrenyl, peryenyl, pyrenyl, fluorenyl, and the like. Particularly, when at least one of A₇ to A₁₂ is N, and at least one of A₇' to A₁₂' is N, one or more carbons of the polycyclic aryls may be substituted with N.

L and L' are the same or different, and are each independently a substituted or unsubstituted C6 to C50 arylene. More particularly, the aryl may be a C6 to C50 aryl, and the aryl may be monocyclic aryls such as phenyl; or or polycyclic aryls such as naphthyl, anthracenyl, phenanthrenyl, pyrenyl, peryenyl, and so on. The compound may be useful for a material of an emission layer.

b and b' are the same or different, and are each independently integers ranging from 1 to 3, and when b and b' are 2 or more, each repeating unit may be different.

When R₁ to R₁₄ in Chemical Formula 112 and R₂, R₄ to R₁₄, and R₇' to R₁₂' in Chemical Formula 113 are a substituted or unsubstituted aryl, a substituted or unsubstituted arylene, or a substituted or unsubstituted arylamine, the aryl may be a C6 to C50 aryl. Particularly, when the aryls are monocyclic aryls such as phenyl, biphenyl, terphenyl, styrene, and so on, or polycyclic aryls such as naphthyl, anthracenyl, phenanthrenyl, pyrenyl, peryenyl, and so on, the compound may be useful for a material of an emission layer.

When R₁ to R₁₄ in Chemical Formula 112 and R₂, R₄ to R₁₄, and R₇' to R₁₂' in Chemical Formula 113 are a substituted or unsubstituted heteroarylamine, or a substituted or unsubstituted heteroaryl, the aryl may be the same as described above. The heteroaryl may refer to an aryl including 1 to 3 heteroatoms of N, O, S, or P, and remaining carbons:

R₁ to R₁₄ in Chemical Formula 112 and R₂, R₄ to R₁₄, and R₇' to R₁₂' in Chemical Formula 113 is a substituted or unsubstituted arylamine, the arylamine may be selected from the group consisting of diphenyl amine, dinaphthyl amine, dibiphenyl amine, phenyl naphthyl amine, phenyl diphenyl amine, ditolyl amine, phenyl tolyl amine, carbazolyl, and triphenyl amine, which provides balance between electron and hole mobility characteristics.

When R₁ to R₁₄ in Chemical Formula 112 and R₂, R₄ to R₁₄, and R₇' to R₁₂' in Chemical Formula 113 are a substituted or unsubstituted heterocycle, the heterocycle the heterocycle is selected from the group consisting of thiophenyl, furanyl, pyrrolyl, imidazolyl, thiazolyl, oxazolyl, oxadiazolyl, triazolyl, pyridinyl, pyridazinyl, quinolinyl, isoquinolinine, acridyl, imidazopyridinyl, and imidazopyrimidinyl.

Particularly, a substituent linked to nitrogen (N) of the imidazolyl or triazolyl is selected from the group consisting of a substituted or unsubstituted alkyl such as a substituted or unsubstituted methyl, a substituted or unsubstituted ethyl, a substituted or unsubstituted propyl, a substituted or unsubstituted isopropyl, a substituted or unsubstituted butyl, a substituted or unsubstituted t-butyl, a substituted or unsubstituted pentyl, a substituted or unsubstituted hexyl, and a substituted or unsubstituted heptyl; a substituted or unsubstituted cycloalkyl such as a substituted or unsubstituted cyclopentyl, a substituted or unsubstituted cyclohexyl, and so on; a substituted or unsubstituted aryl such as a substituted or unsubstituted phenyl, a substituted or unsubstituted biphenyl, a substituted or unsubstituted naphthyl, and so on; and a substituted or unsubstituted heterocycle. The heterocycle is preferably a heteroaryl such as pyridyl, bipyridyl, quinolyl, isoquinolyl, and so on.

At least one of R₁ to R₁₂ in Chemical Formula 112 and at least one of R₂, R₄ to R₁₂, and R₇' to R₁₂' in Chemical Formula 113 are substituted with at least one of substituent selected from an amine-substituted alkyl, an amine-substituted cycloalkyl, an amine-substituted aryl, and an amine-substituted heterocycle, which makes the compound be applicable to a hole injection layer (HIL) or a hole transport layer (HTL).

At least one R₁ to R₁₂ in Chemical Formula 112 and at least one of R₂, R₄ to R₁₂, R₇' to R₁₂' in Chemical Formula 113 is substituted with a substituent selected from the group consisting of a nitrile, a nitro, an amide, a carbonyl, and a substituted or unsubstituted heterocycle, which reinsforces an electron injection or transport capability and thereby makes the compound be applicable to an electron injection layer (EIL) or an electron transport layer (ETL). Accordingly, hole transport and electron transport capibilties may be simultaneously improved.

The various substituents of the above-described compound represented by the above Formulae 112 and 113 do not change principal properties of the compound for an organic photoelectric device according to one embodiment.

Examples of the compounds for an organic photoelectric device according to one embodiment of the present invention include the compounds represented by the following Formulae, but are not limited thereto.

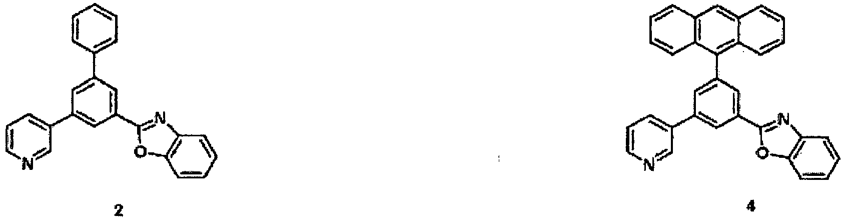

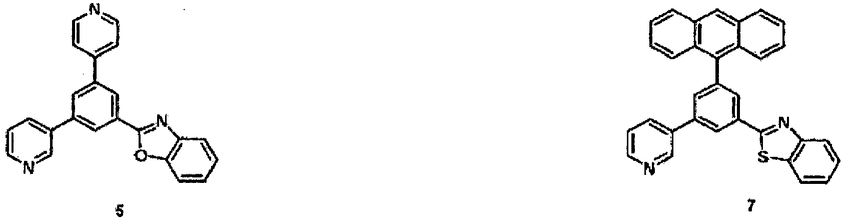

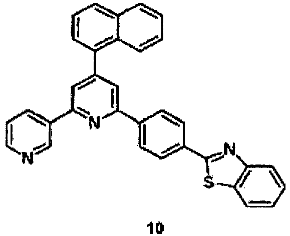

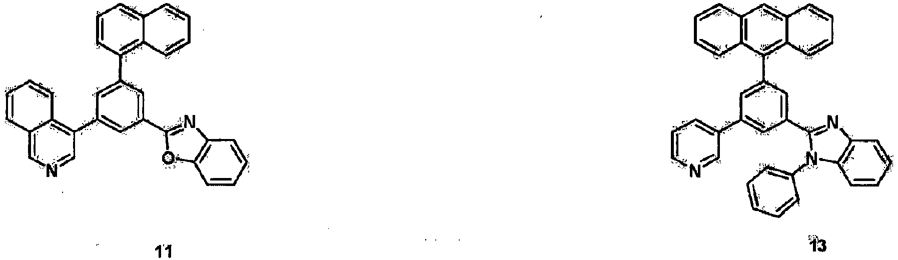

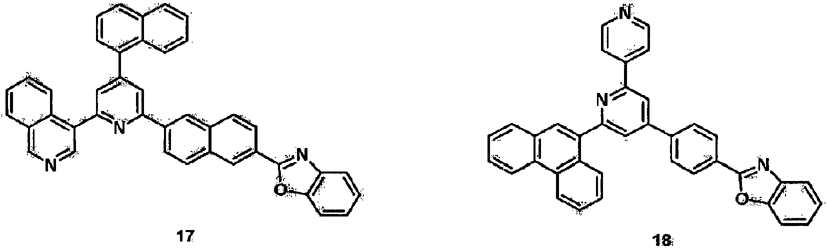

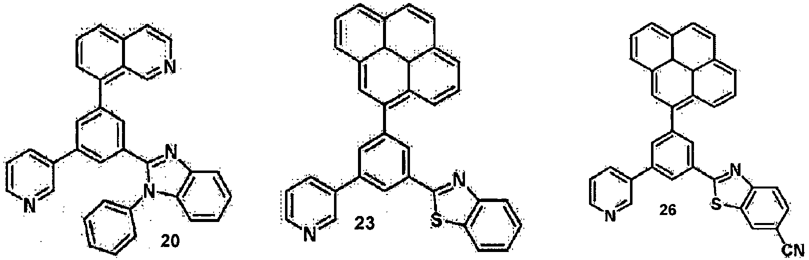

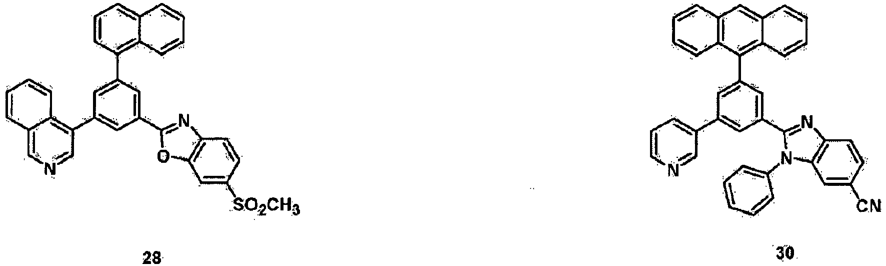

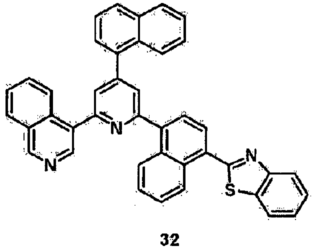

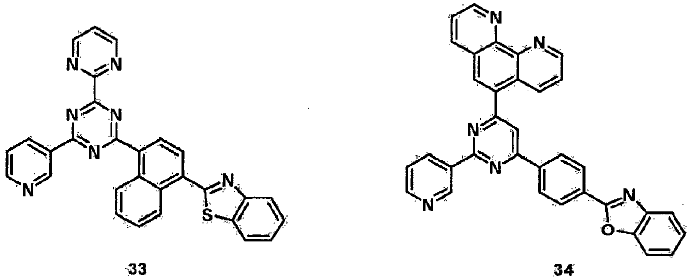

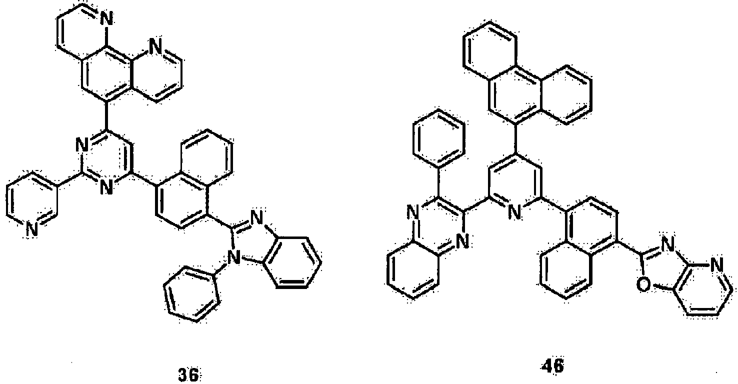

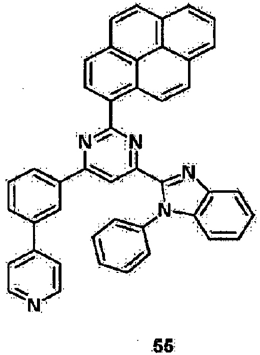

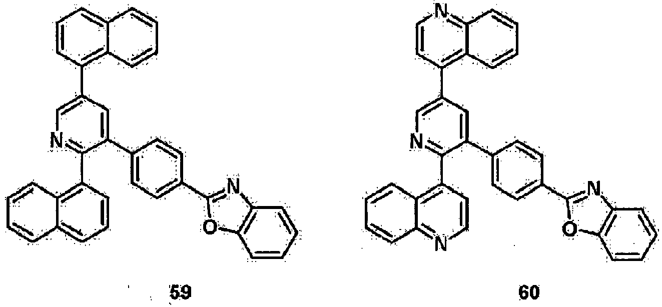

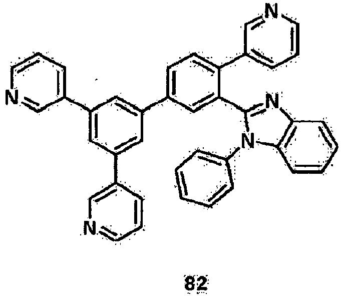

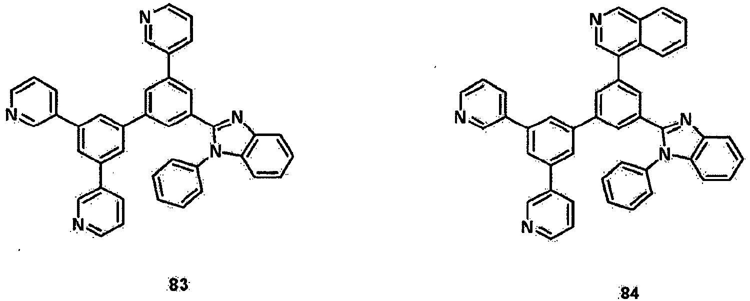

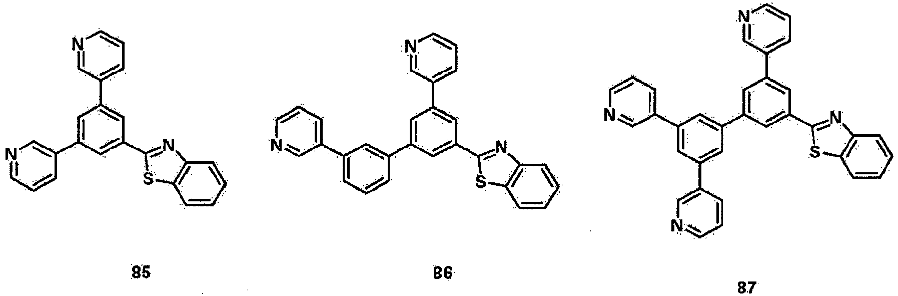

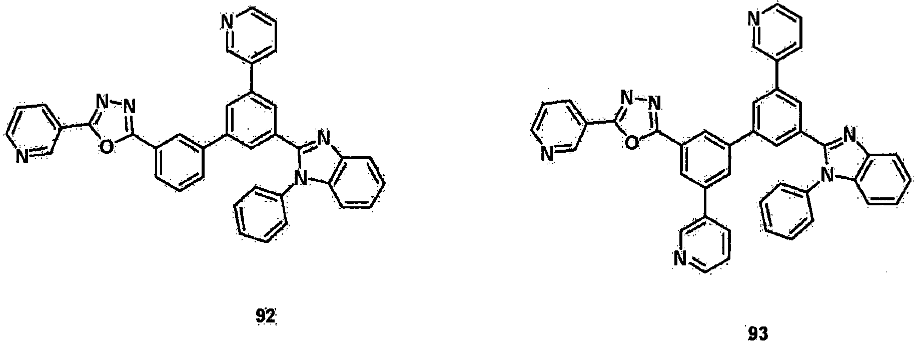

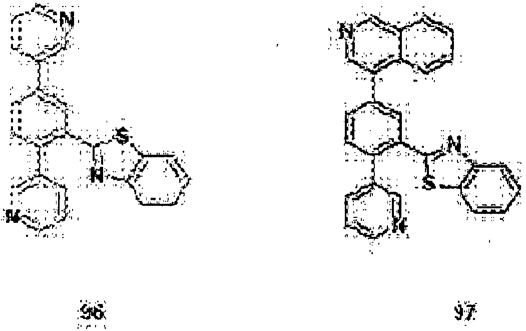

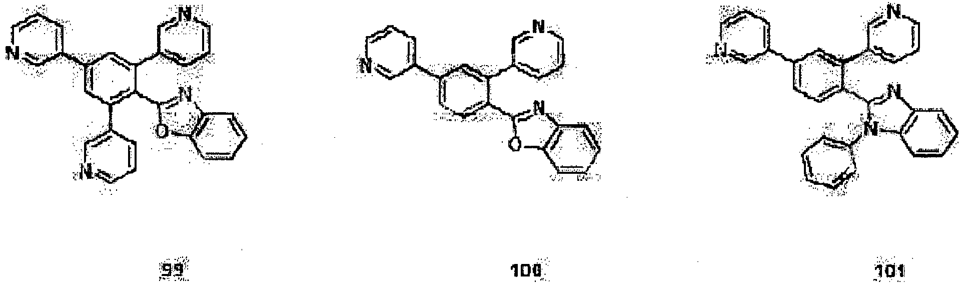

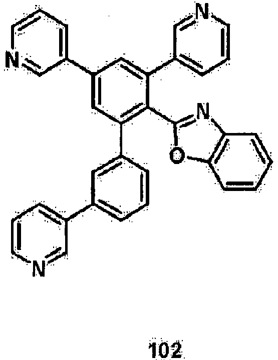

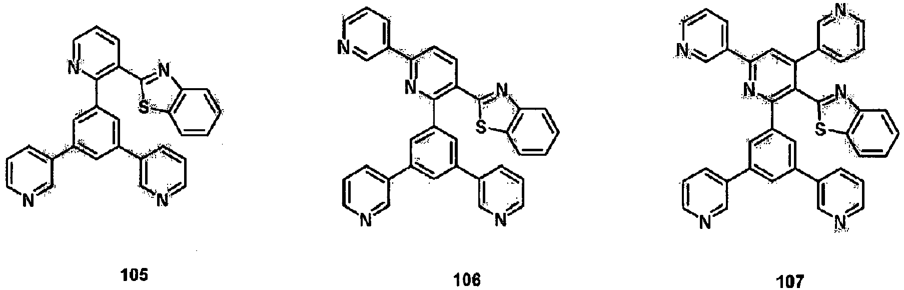

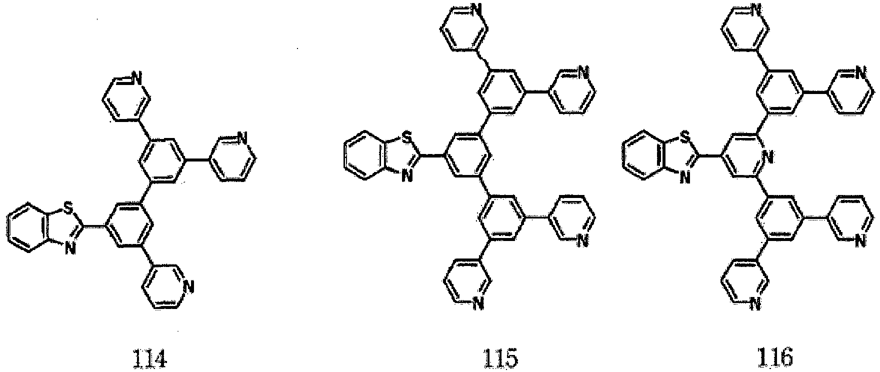

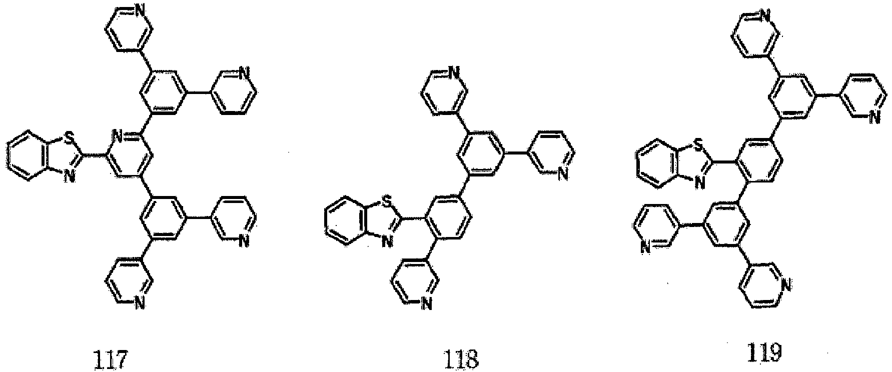

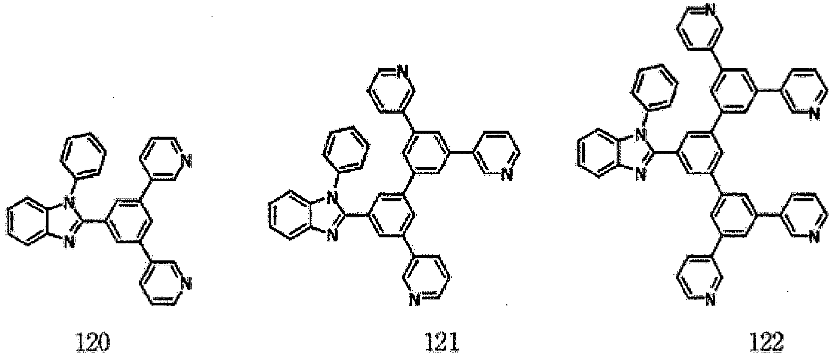

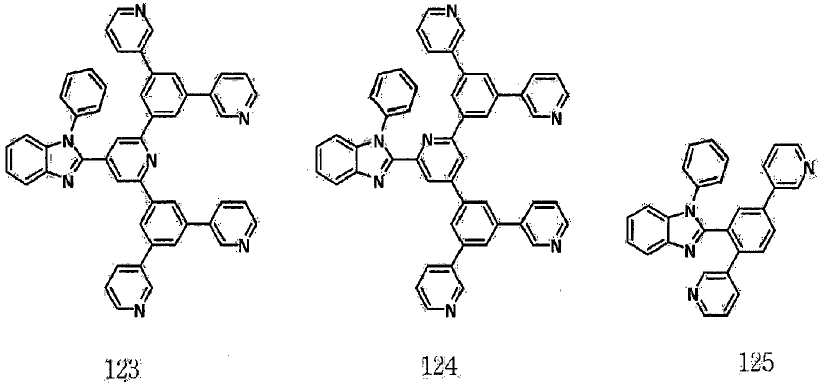

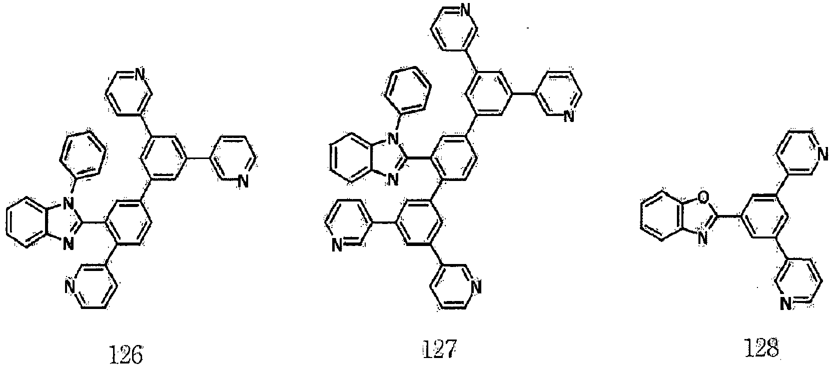

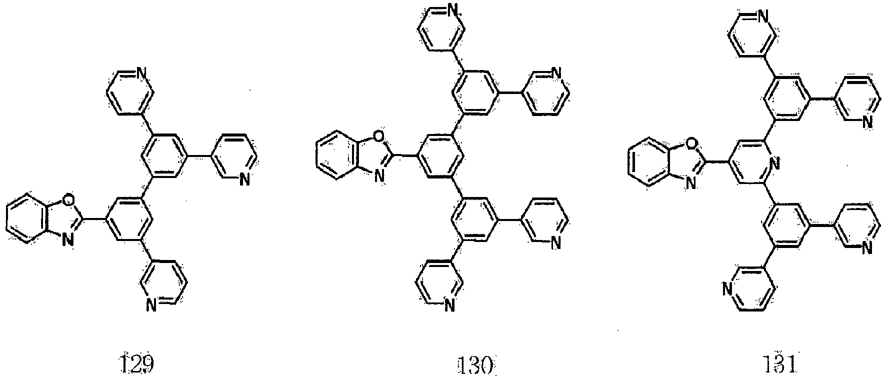

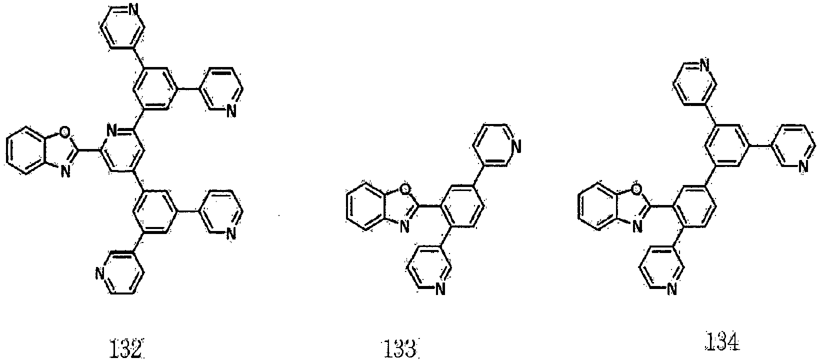

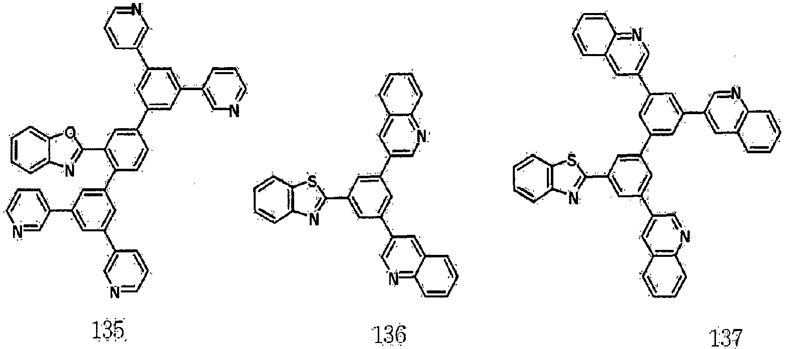

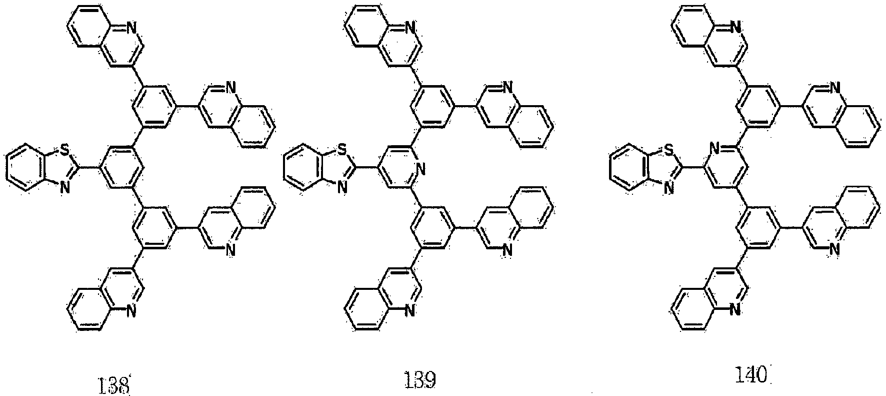

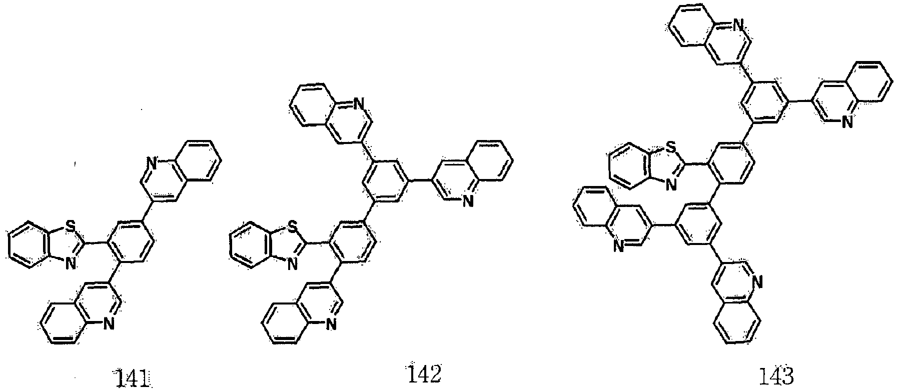

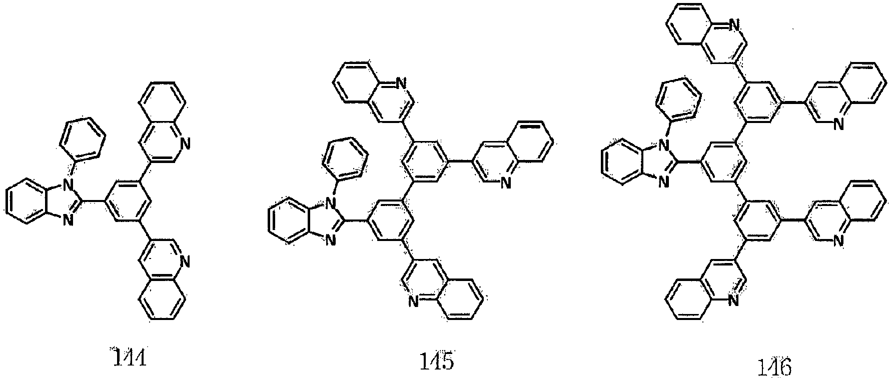

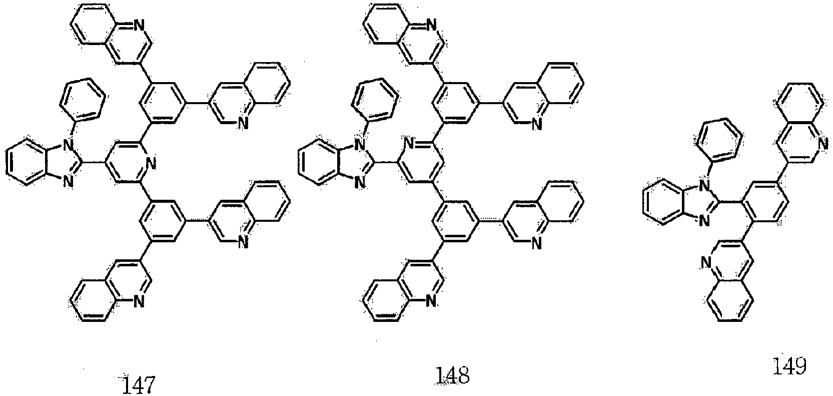

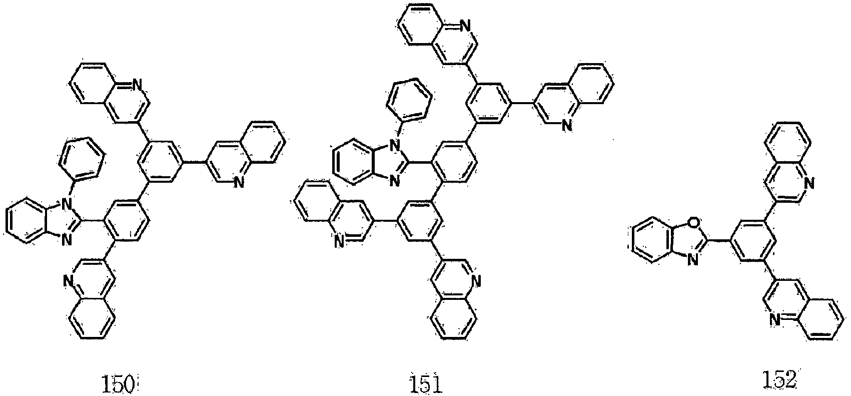

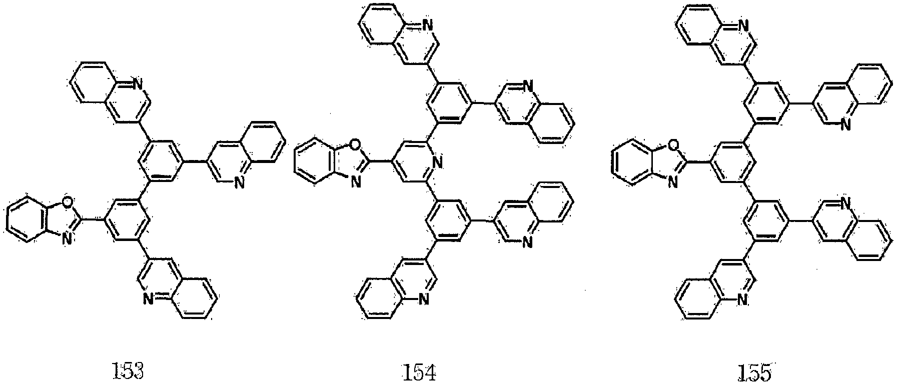

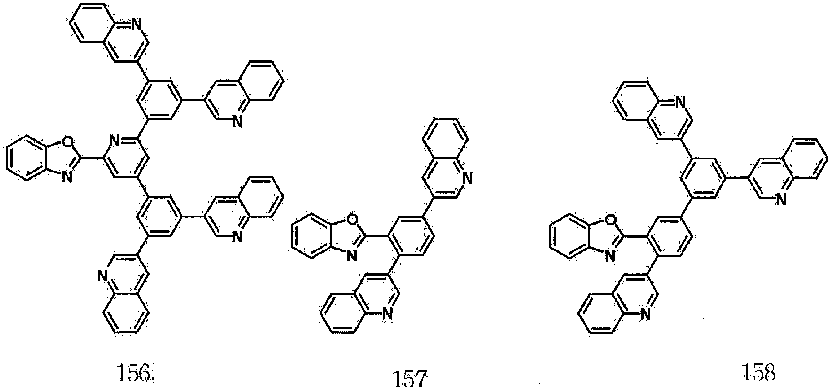

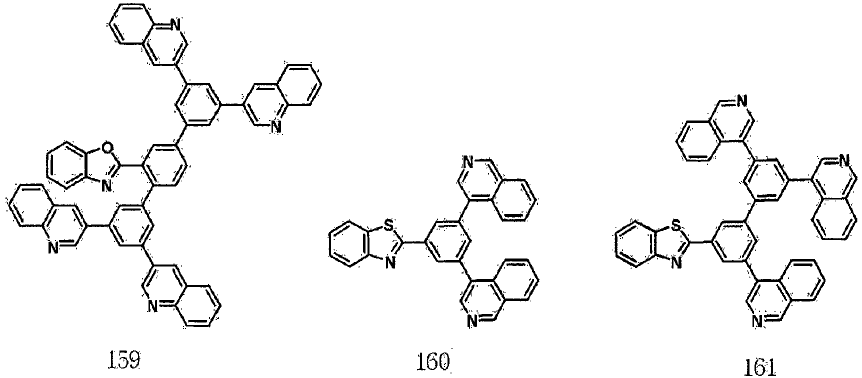

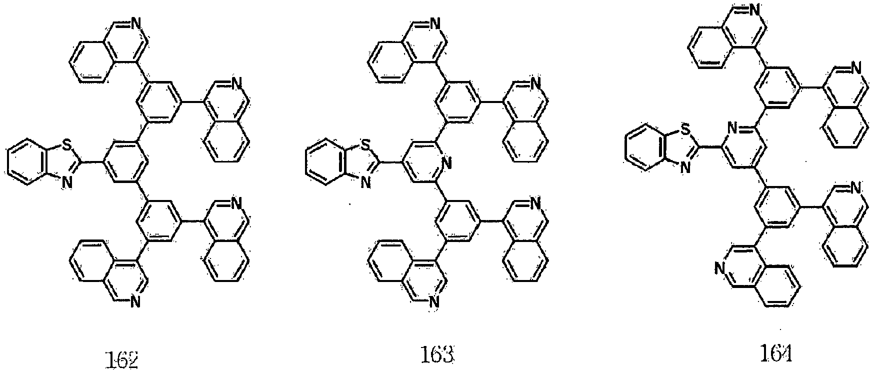

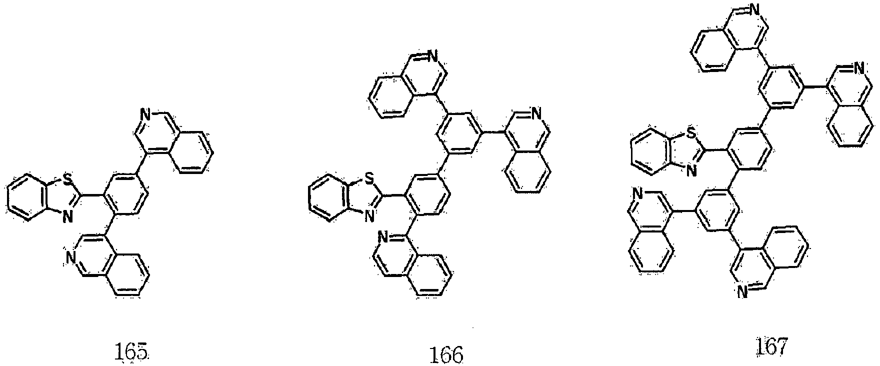

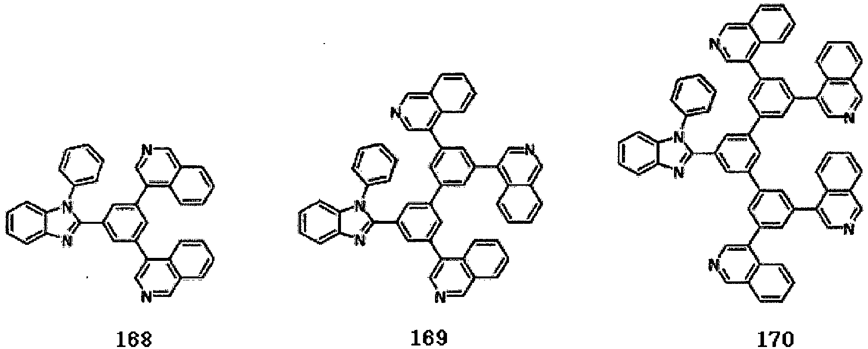

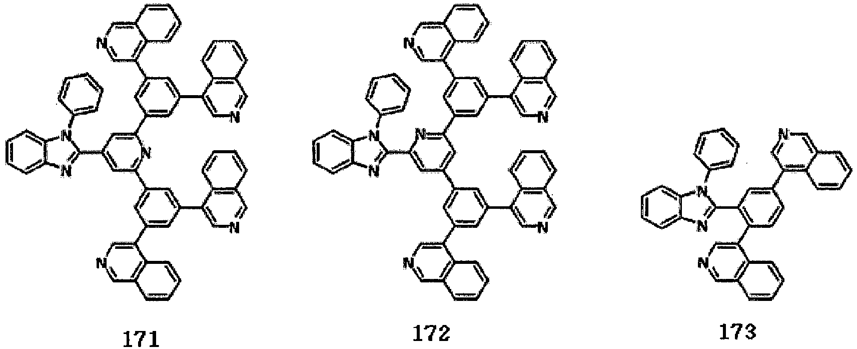

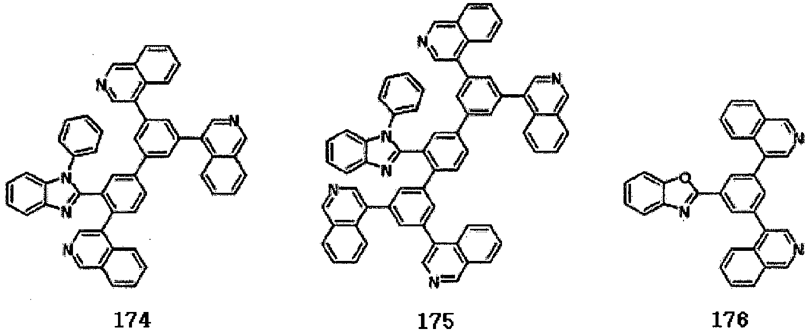

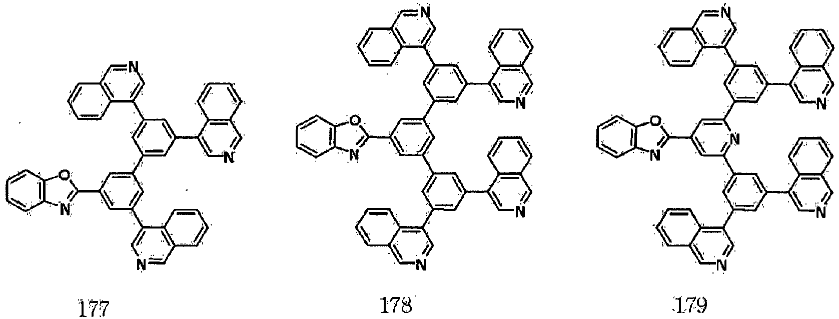

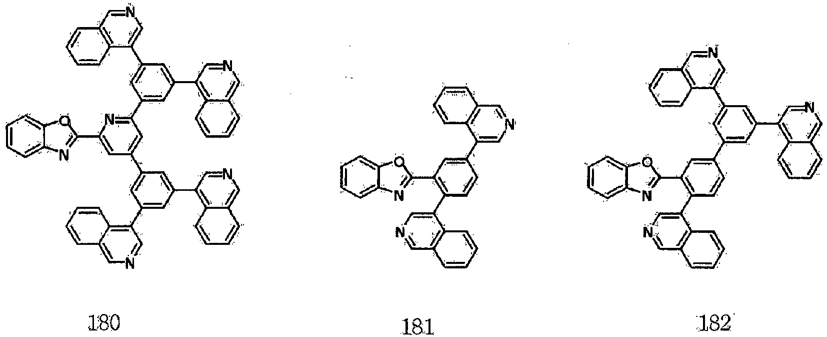

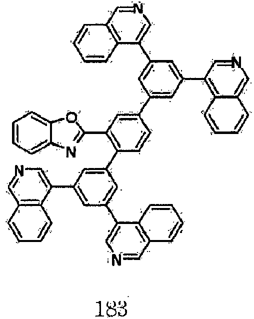

A compound for an organic photoelectric device including the above compound can play a role of hole injection, hole transport, light emitting, or electron injection and/or transport, and also as a light emitting host with an appropriate dopant. The compound for an organic photoelectric device can improve thermal stability and decrease a driving voltage for improving life-span and efficiency characteristics of an organic photoelectric device when included in an organic thin layer.

The compound for an organic photoelectric device including the above compound can play a role of hole and electron transport, and also as a light emitting host with an appropriate dopant. Particularly, the dopant may be a reducing dopant. The reducing dopant is selected from the group consisting of an alkaline metal, an alkaline earth metal, a rare earth element metal, an oxide of an alkaline metal, a halide of an alkaline metal, an organic complex of an alkaline metal, an oxide of an alkaline earth metal, a halide of an alkaline earth metal, an organic complex of an alkaline earth metal, an oxide of an alkaline earth metal, a halide of an alkaline earth metal, an organic complex of an alkaline earth metal, an oxide of a rare earth element metal, a halide of a rare earth element metal, and an organic complex of a rare earth element metal.

The new compound for an organic photoelectric device can be usefully used for a host material or a charge transfer material. Herein, the organic photoelectric device may include an organic light emitting diode, an organic solar cell, an organic transistor, an organic memory device, and the like.

As for an organic solar cell, the new compound of the present invention is included in an electrode or an electrode buffer layer and can thereby improve quantum efficiency. As for an organic transistor, it can be used as an electrode material in a gate, a source-drain electrode, and the like.

Hereinafter, an organic light emitting diode is illustrated in more detail. An organic photoelectric device according to another embodiment of the present invention includes the compound for an organic photoelectric device in at least one layer among organic thin layers, when an organic photoelectric device in general includes an anode, a cathode, and at least one organic thin layer disposed between the anode and cathode.

An organic light emitting diode includes a new compound having various energy band gaps, and thereby satisfies various conditions required for a hole injection layer (HIL), a hole transport layer (HTL), an emission layer, an electron injection layer (EIL), an electron transport layer (ETL), and the like. Accordingly, the organic light emitting diode can realize a low driving voltage and high luminous efficiency.

The organic thin layer can include one selected from the group consisting of an emission layer, an electron transport layer (ETL), an electron injection layer (EIL), a hole transport layer (HTL), a hole injection layer (HIL), and a hole blocking layer. At least one layer includes a compound for an organic photoelectric device according to the present invention.

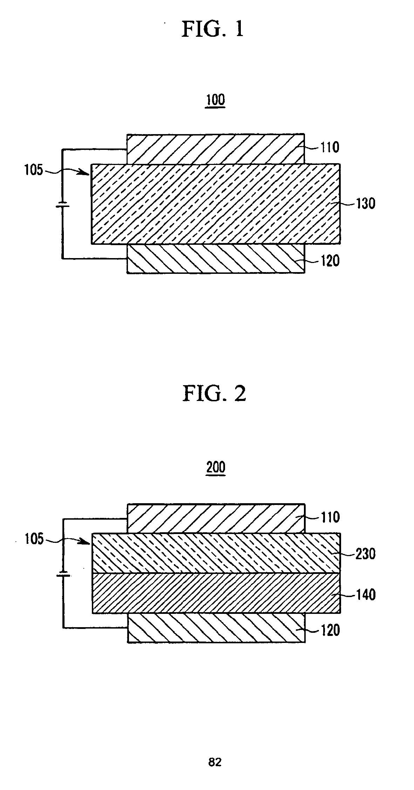

FIGS. 1 to 5 are cross-sectional views showing organic photoelectric devices including the novel organic compounds according to various embodiments of the present invention.

Referring to FIGS. 1 to 5, the organic photoelectric devices 100, 200, 300, 400, and 500 according to one embodiment includes at least one organic thin layer 105 interposed between an anode 120 and a cathode 110.

The anode 120 includes an anode material laving a large work function to help hole injection into an organic thin layer. The anode material includes a metal such as nickel, platinum, vanadium, chromium, copper, zinc, and gold, or alloys thereof; a metal oxide such as zinc oxide, indium oxide, indium tin oxide (ITO), and indium zinc oxide (IZO); a combined metal and oxide such as ZnO:Al or SnO₂:Sb; or a conductive polymer such as poly(3-methylthiophene), poly[3,4-(ethylene-1,2-dioxy)thiophene] (PEDT), polypyrrole, and polyaniline, but is not limited thereto.

The cathode 110 includes a cathode material having a small work function to help electron injection into an organic thin layer. The cathode material includes a metal such as magnesium, calcium, sodium, potassium, titanium, indium, yttrium, lithium, gadolinium, aluminum, silver, tin, and lead, or alloys thereof; or a multi-layered material such as LiF/Al, Liq/Al, LiO₂/Al, LiF/Ca, LiF/Al, and BaF₂/Ca, but is not limited thereto.

Referring to FIG. 1, the organic photoelectric device 100 includes an organic thin layer 105 including only an emission layer 130.

Referring to FIG. 2, a double-layered organic photoelectric device 200 includes an organic thin layer 105 including an emission layer 230 including an electron transport layer (ETL), and a hole transport layer (HTL) 140. The emission layer 130 also functions as an electron transport layer (ETL), and the hole transport layer (HTL) 140 layer has an excellent binding property with a transparent electrode such as ITO or an excellent hole transporting property.

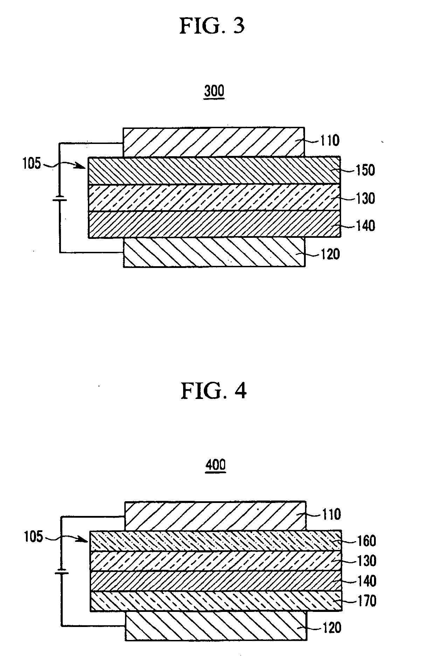

Referring to FIG. 3, a three-layered organic photoelectric device 300 includes an organic thin layer 105 including an electron transport layer (ETL) 150, an emission layer 130, and a hole transport layer (HTL) 140. The emission layer 130 is independently installed, and layers having an excellent electron transporting property or an excellent hole transporting property are separately stacked.

As shown in FIG. 4, a four-layered organic photoelectric device 400 includes an organic thin layer 105 including an electron injection layer (EIL) 160, an emission layer 130, a hole transport layer (HTL) 140, and a hole injection layer (HIL) 170 for binding with the cathode of ITO.

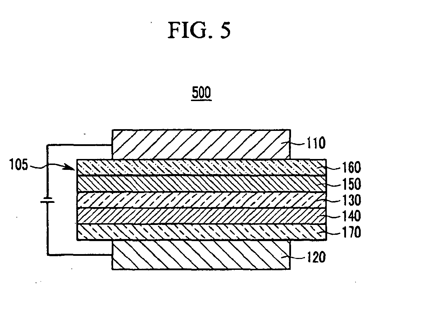

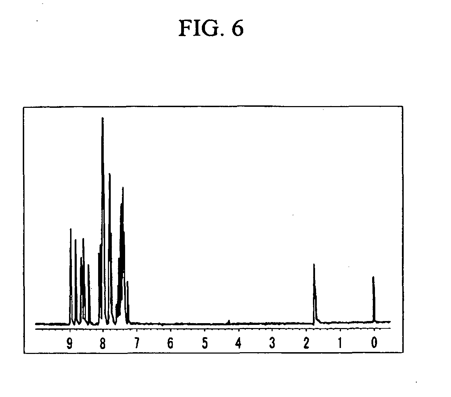

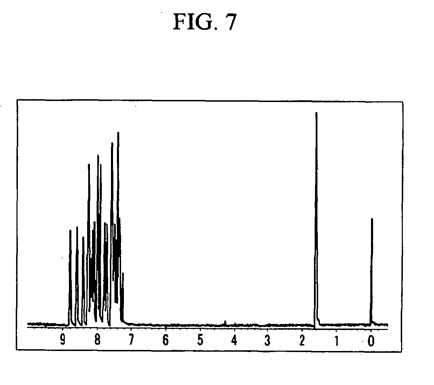

As shown in FIG. 5, a five layered organic photoelectric device 500 includes an organic thin layer 105 including an electron transport layer (ETL) 150, an emission layer 130, a hole transport layer (HTL) 140, and a hole injection layer (HIL) 170, and further includes an electron injection layer (EIL) 160 to achieve a low voltage.

The light emitting diode can be fabricated by forming an anode on a substrate, forming an organic thin layer, and forming a cathode thereon. The organic thin layer can be formed by a dry coating method such as evaporation, sputtering, plasma plating, and ion plating, or a wet coating method such as spin coating, dipping, and flow coating.

Another embodiment of the present invention provides a display device including the organic photoelectric device according to the above embodiment.

Hereinafter, the embodiments are illustrated in more detail with reference to examples. However, it is understood that the present invention is not limited by these examples.

### Synthesis Example 3: Synthesis of 2-(3,5-dibromophenyl)-1-phenyl-1H-benzo[d]imidazole

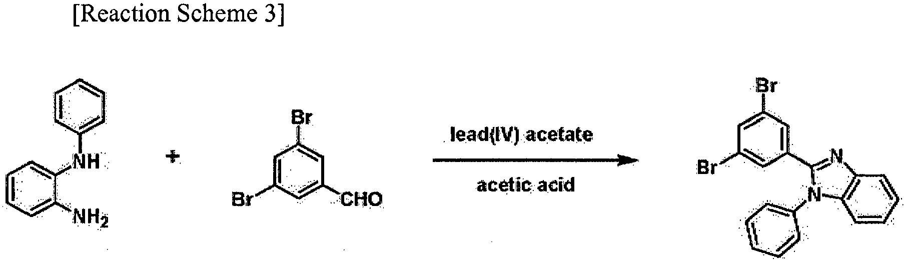

20 g (75.8 mmol) of 3,5-dibromobenzaldehyde and 16.8 g (90.9 mmol) of N-phenyl-o-phenylenediamine were dissolved in 150 mL of acetic acid. The solution was agitated at room temperature for 30 minutes. Next, 37 g (83.4 mmol) of lead(IV) acetate was added thereto. The resulting product was agitated at 50°C for 1 hour. Then, water was poured into the obtained reactant. The resulting product was treated with ethylacetate to perform extraction and the solvent was removed under a reduced pressure. The extract was separated through a column and dried, obtaining 9.5 g (Y=29 %) of a yellow solid.

### Synthesis Example 4: Synthesis of 2-(3-bromo-5-(pyridin-3-yl)phenyl)-1-phenyl-1H-benzo[d]imidazole

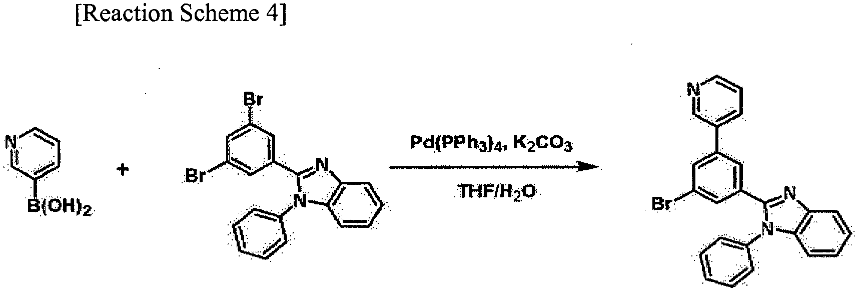

9.4 g (22.0 mmol) of 2-(3,5-dibromophenyl)-1-phenyl-1H-benzo[d]imidazole according to Synthesis Example 3, 2.7 g (22.0 mmol) of pyridine-3-boronic acid, 0.76 g (3 mol%) of tetrakis(triphenylphosphine)palladium (0), and 6.1 g (44.0 mmol) of potassium carbonate were dissolved in 300 ml of tetrahydrofuran/ H₂O mixed in a volume ratio of 2/1. The solution was reacted at 80°C for 12 hours. The obtained reactant was extracted with ethylacetate and treated under a reduced pressure to remove the solvent therein. The reactant was separated through a column and dried, obtaining 5.15 g (Y=54 %) of a yellow solid.

### Synthesis Example 6: Synthesis of 2-(3-bromo-5-(pyridin-3-yl)phenyl)benzo[d]thiazole

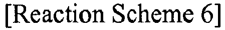

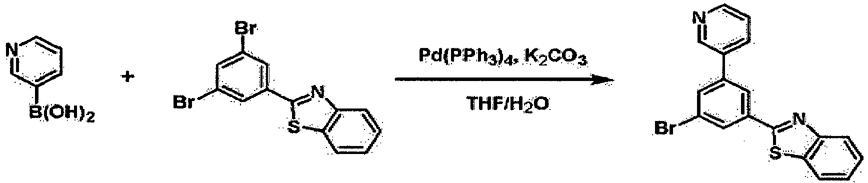

4.74 g (12.8 mmol) of 2-(3,5-dibromophenyl)benzo[d]thiazole) according to Synthesis Example 5, 1.89 g (15.4 mmol) of pyridine-3-boronic acid, 0.44 g (3 mol%) of tetrakis(triphenylphosphine)palladium (0), and 5.31 g (38.4 mmol) of potassium carbonate were dissolved in 50 ml of tetrahydrofuran/H₂O mixed in a volume ratio of 4/1. The solution was reacted at 80°C for 12 hours. The obtained reactant was extracted with ethylacetate and treated under a reduced pressure to remove the solvent. The extract was separated through a column and dried, obtaining 2.54 g (Y=54 %) of a white so lid.

### Synthesis Example 7: Synthesis of 3-(2H-tetrazol-5-yl)pyridine

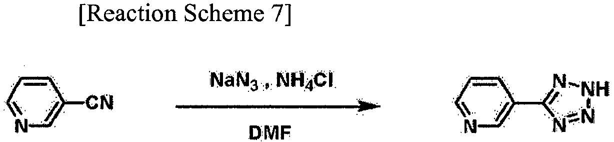

30 g (288 mmol) of 3-pyridinecarbonitrile, 28.1 g (432 mmol) of sodium azide (NaN₃), and 23.1 g (432 mmol) of ammonium chloride were dissolved in 200 mL of DMF. The solution was reacted at 100°C for 24 hours. Then, water was added to the obtained reactant. The resulting product was neutralized with hydrochloric acid and then fitered, obtaining 19.6 g (Y=46 %) of a white solid.

### Synthesis Example 8: Synthesis of 3-(5-(3-bromophenyl)-1,3,4-oxadiazol-2-yl)pyridine

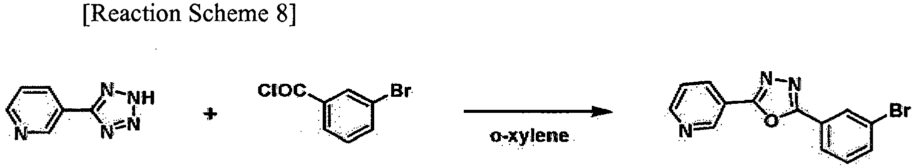

16.8 g (114 mmol) of 3-(2H-tetrazol-5-yl)pyridine) according to Synthesis Example 7 and 25 g (114 mmol) of 3-bromobenzoylchloride were dissolved in 180 mL of o-xylene. The solution was reacted at 150°C for 8 hours. The obtained reactant was purified under a reduced pressure and washed with methanol, obtaining 30 g (Y=87 %) of a white solid.

### Synthesis Example 9: Synthesis of 3-(5-(3-(4,4,5,5-tetramethyl-1,3,2-dioxaborolan-2-yl)phenyl)-1,3,4-oxadiazol-2-yl)pyridine

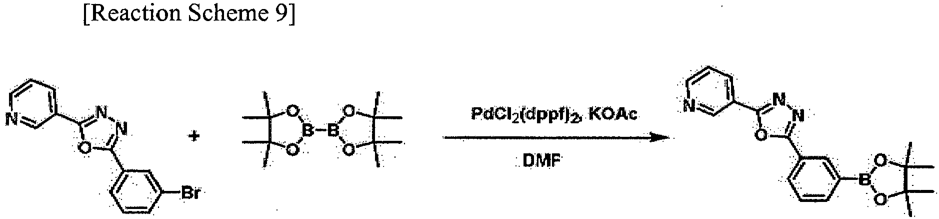

12 g (40 mmol) of 3-(5-(3-bromophenyl)-1,3,4-oxadiazol-2-yl)pyridine according to Synthesis Example 8, 12.2 g (48 mmol) of bis(pinacolato)diboron, 0.98 g (3 mol%) of a complex of [1,1'-bis(diphenylphosphino)ferrocene]dichloro-palladium(II) with dichloromethane, 5.9 g (60 mmol) of potassium acetate were dissolved in 250 ml of dimethylformaimde (DMF). The solution was reacted at 80 °C for 12 hours. The obtained reactant was extracted with ethylacetate and treated under a reduced pressure to remove the solvent. The extract was separated through a column and dried, obtaining 10 g (Y=71 %) of a white solid.

### Synthesis Example 10: Synthesis of 2-(2,5-dibromophenyl)benzo[d]thiazole

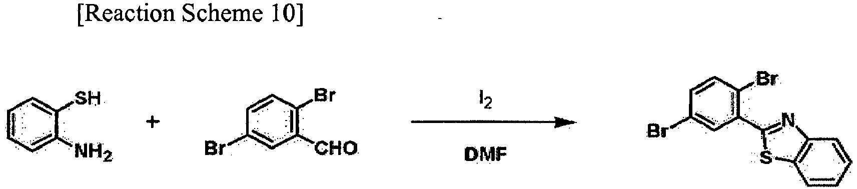

5 g (18.5 mmol) of 2,5-dibromobenzaldehyde, 3 mL (27.7 mmol) of 2-aminothiophenol, and 2.35 g (9.25 mmol) of iodine were dissolved in 100 mL of DMF. The solution was reacted at 100°C for 1 hour. The obtained reactant was purified through a column, obtaining 4.64 g (Y=68 %) of a white solid.

### Synthesis Example 11: Synthesis of 2-(2-bromopyridin-3-yl)benzo[d]thiazole

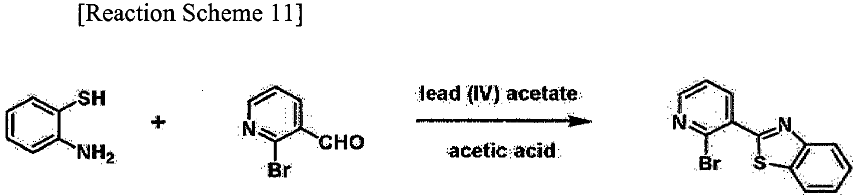

6.58 g (34.0 mmol) of 2-bromo-3-pyrdinecarboxaldehyde and 5.5 mL (50.9 mmol) of 2-aminothiophenol were dissolved in 150 mL of acetic acid. The solution was agitated at room temperature for 30 minutes. Next, 19.0 g (40.8 mmol) of lead (IV) acetate was added thereto. The resulting product was agitated at 50°C for 1 hour. Then, water was added to the obtained reactant. The mixture was extracted with ethylacetate and treated under a reduced pressure to remove the solvent, obtaining 5.55 g (Y=55 %) of a white solid.

### Example 3: Synthesis of a compound of Chemical Formula 83

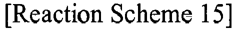

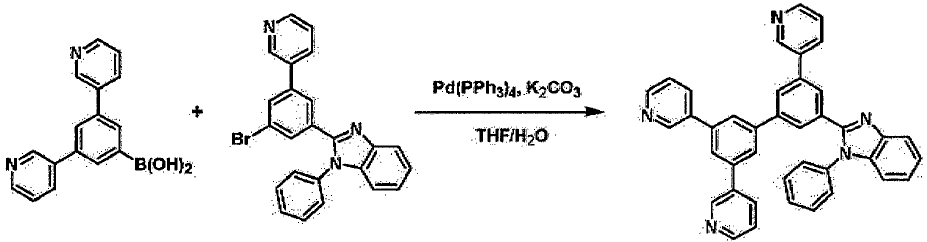

4.14 g (15.0 mmol) of 3,5-di-(3-pyridinyl)-benzene boronic acid, 6.4 g (15.0 mmol) of 2-(3-bromo-5-(pyridin-3-yl)phenyl)-1-phenyl-1H-benzo[d]imidazole according to Synthesis Example 4, 0.52 g (3 mol%) of tetrakis(triphenylphosphine)palladium (0), and 4.15 g (30.0 mmol) of potassium carbonate were dissolved in 300 ml of a solvent of tetrahydrofuran/H₂O mixed in a volume ratio of 2/1. The solution was reacted at 80°C for 12 hours. The obtained reactant was extracted with ethylacetate and treated under a reduced pressure to remove the solvent. The extract was separated through a column and dried, obtaining 1.2 g (Y=13 %) of a white solid.

### Example 4: Synthesis of a compound of Chemical Formula 87

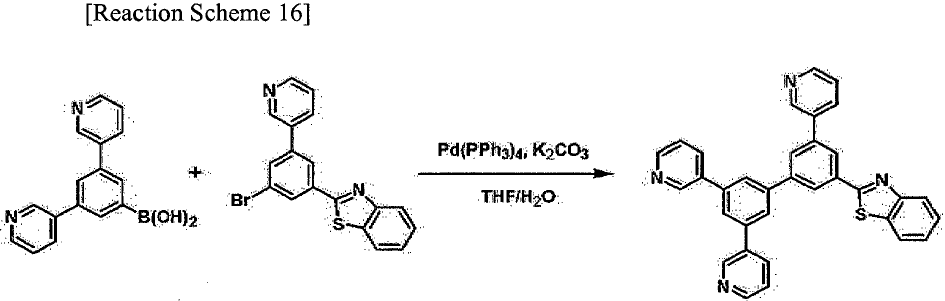

0.84 g (3.1 mmol) of 3,5-di-(3-pyridinyl)-benzene boronic acid), 1.0 g (2.8 mmol) of 2-(3-bromo-5-(pyridin-3-yl)phenyl)benzo[d]thiazole according to Synthesis Example 6, 0.14 g (5 mol%) of tetrakis(triphenylphosphine)palladium (0), and 1.15 g (8.34 mmol) of potassium carbonate were dissolved in 25 mL of a solvent of tetrahydrofuran/H₂O mixed in a volume ratio of 4/1. The solution was reacted at 80°C for 12 hours. The obtained reactant was extracted with ethylacetate and treated under a reduced pressure to remove the solvent. The extract was separated through a column and dried, obtaining 1.35 g (Y=92 %) of a white solid.

### Example 5: Synthesis of a compound of Chemical Formula 92

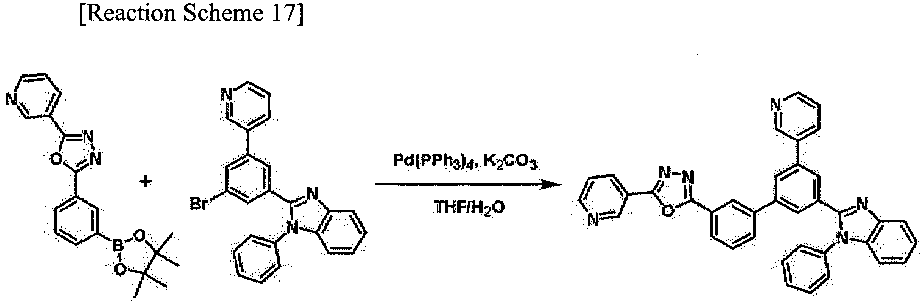

4.63 g (13.3 mmol) of 3-(5-(3-(4,4,5,5-tetramethyl-1,3,2-dioxaborolan-2-yl)phenyl)-1,3,4-oxadiazol-2-yl)pyrid ine according to Synthesis Example 9, 5.15 g (12.1 mmol) of 2-(3-bromo-5-(pyridin-3-yl)phenyl)-1-phenyl-1H-benzo[d]imidazole) according to Synthesis Example 4, 0.47 g (3 mol%) of tetrakis(triphenylphosphine)palladium (0), and 3.3 g (24.2 mmol) of potassium carbonate were dissolved in 300 mL of a solvent tetrahydrofuran/H₂O mixed in a volume ratio of 4/1. The solution was reacted at 80°C for 12 hours. The obtained reactant was extracted with ethylacetate and treated under a reduced pressure to remove the solvent. The extract was separated through a column and dried, obtaining 2 g (Y=33 %) of a white solid.

### Example 6: Synthesis of a compound of Chemical Formula 96

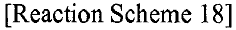

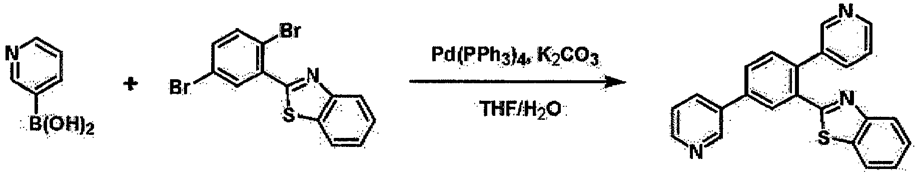

1 g (2.76 mmol) of 2-(2,5-dibromophenyl)benzo[d]thiazole according to Synthesis Example 10, 0.85 g (6.9 mmol) of pyridine-3-boronic acid, 0.28 g (10 mol%) of tetrakis(triphenylphosphine)palladium (0), and 2.3 g (16.6 mmol) of potassium carbonate were dissolved in 25 mL of a solvent of tetrahydrofuran/H₂O mixed in a volume ratio of 4/1. The solution was reacted at 80°C for 12 hours. The obtained reactant was extracted with ethylacetate and treated under a reduced pressure to remove the solvent. The extract was separated through a column and dried, obtaining 0.8 g (Y=79 %) of a white solid.

### Example 7: Synthesis of a compound of Chemical Formula 105

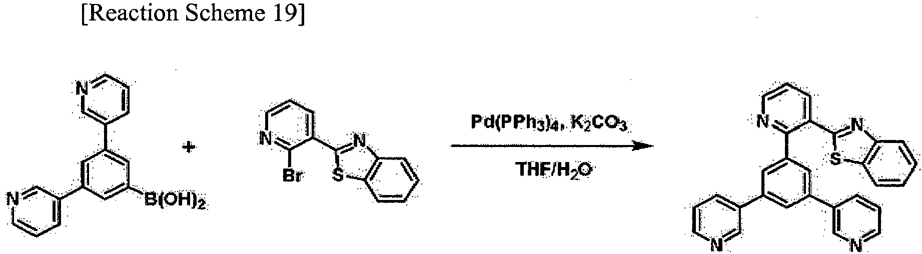

1.33 g (4.8mmol) of 3,5-di-(3-pyridinyl)-benzene boronic acid, 1.7 g (5.8 mmol) of 2-(2-bromopyridine-3-yl)benzo[d]thiazole according to Synthesis Example 11, 0.28 g (5 mol%) of tetrakis(triphenylphosphine)palladium (0), and 2.0 g (14.4 mmol) of potassium carbonate were dissolved in 30 mL of a solvent of tetrahydrofuran/H₂O mixed in a volume ratio of 4/1. The solution was reacted at 80°C for 12 hours. The obtained reactant was extracted with ethylacetate and treated under a reduced pressure to remove the solvent. The extract was separated through a column and dried, obtaining 2.28 g (Y=88 %) of a white solid.

### Comparative Example 1: 2-(4-biphenylyl)-5- (4-t-butylphenyl)-1,3,4-oxadiazole (PBD)

2-(4-biphenylyl)-5-(4-t-butylphenyl)-1,3,4-oxadiazole (PBD), which is conventionally known to have an excellent charge transport capability (Jpn. J. Appl. Phys., 27, L269 1988), was prepared.

On the electron transport layer (ETL), LiF 0.5 nm/A1 100 nm were respectively and sequentially vacuum-deposited to form a cathode including LiF/Al, fabricating an organic light emitting diode of Example 10.

### Example 11: Fabrication of an organic light emitting diode

An organic light emitting diode was fabricated according to the same method as in Example 10, except for using a compound according to Example 3 to form an electron transport layer (ETL).

### Example 12: Fabrication of an organic light emitting diode

An organic light emitting diode was fabricated according to the same method as in Example 10, except for using a compound according to Example 4 to form an electron transport layer (ETL).

### Example 13: Fabrication of an organic light emitting diode

An organic light emitting diode was fabricated according to the same method as in Example 10, except for using a compound according to Example 5 to form an electron transport layer (ETL).

### Example 14: Fabrication of an organic light emitting diode

An organic light emitting diode was fabricated according to the same method as in Example 10, except for using a compound according to Example 7 to form an electron transport layer (ETL).

### Comparative Example 2: Fabrication of an organic light emitting diode

An organic light emitting diode was fabricated according to the same method as in Example 10, except for using an electron transportmaterial of Alq₃ to form an electron transport layer (ETL).

### Property measurement and analysis

### 1) Driving voltage, luminance and luminous efficiency

The organic light emitting diodes of Examples 10 to 16 and Comparative Example 2 were measured regarding a driving voltage (V_{d}) required to produce luminance of 1000 nit, and current efficiency (cd/A) and electrical power efficiency (lm/W) at the same luminance. The results are shown in the following Table 1.

**(Table 1)**

| Devices | At 1000 cd/m² | | | Vₒₙ | Max. | |
|---|---|---|---|---|---|---|
| | V_{d} (V) | cd/A | lm/W | | cd/A | lm/W |
| Comparative Example 2 | 7.40 | 4.68 | 1.99 | 3.60 | 4.72 | 2.88 |
| Example 10 | 6.20 | 5.19 | 2.63 | 3.20 | 5.35 | 3.25 |
| Example 11 | 7.20 | 5.29 | 2.31 | 3.80 | 5.32 | 2.77 |
| Example 12 | 6.40 | 5.00 | 2.45 | 3.00 | 5.32 | 3.34 |
| Example 13 | 7.40 | 4.99 | 2.12 | 3.20 | 5.32 | 3.37 |
| Example 14 | 6.40 | 5.80 | 2.85 | 3.00 | 5.88 | 3.51 |
| | | | | | | |
| | | | | | | |

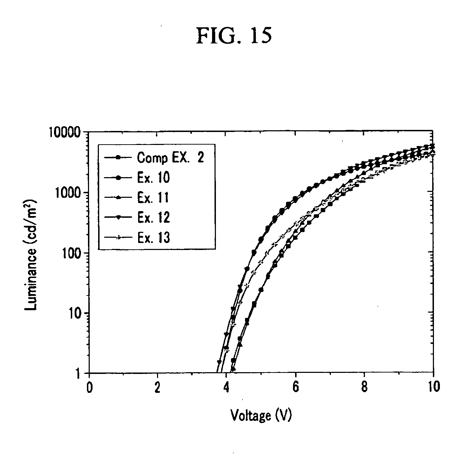

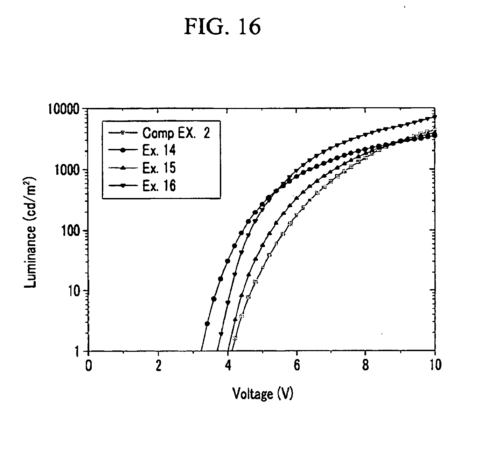

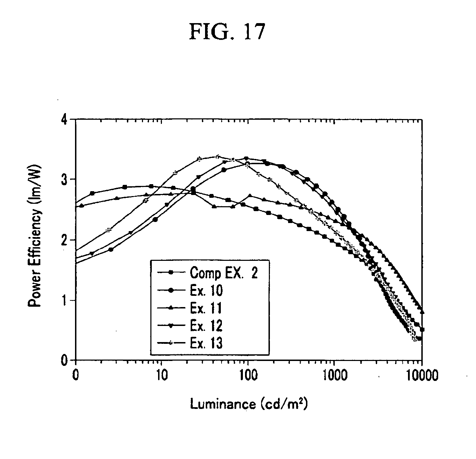

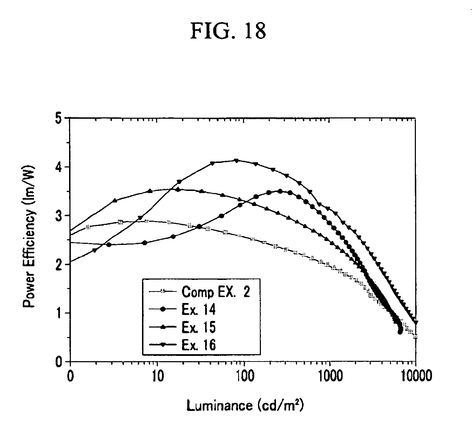

Referring to Table 1 and FIGS. 15 and 16, the organic light emitting diodes of Examples 11 to 14 had a sharply lower driving voltage than those of Comparative Example 2. In addition, referring to Table 1 and FIGS. 17 and 18, the organic light emitting diodes of Examples 11 to 14 had a sharply low driving voltage but superbly high current efficiency and electrical power efficiency.

These measurement results of the organic light emitting diodes come from combination balance of holes and electrons in the emission layer. The compounds of Examples 11 to 14 turned out to have excellent electron injection and transport characteristics compared with Alq₃, a general electron transport material.

### 2) Thermal stability

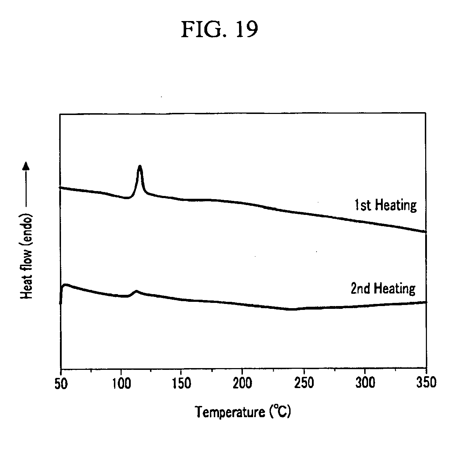

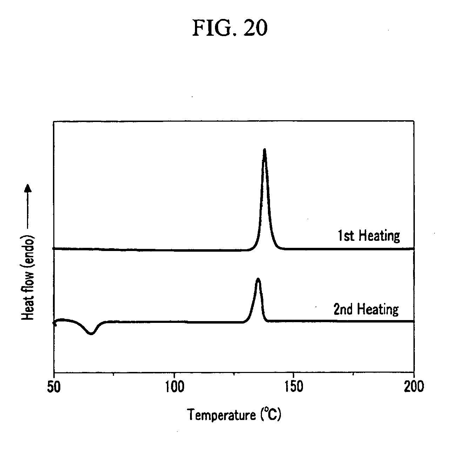

The compounds according to Examples 3 to 7 were analyzed in a differential scanning calorimetry method (DSC), and then secondarily analyzed in the same method. The analysis results of the compounds according to Example 5 and Comparative Example 1 are shown in FIGS. 19 and 20. Referring to FIGS. 19 and 20, the compound of Example 5 had melting point peaks in the primary analysis, but no melting point peak in the secondary analysis. On the contrary, 2-(4-biphenylyl)-5-(4-t-butylphenyl)-1,3,4-oxadiazole (PBD) according to Comparative Example 1 had a melting point peak at 138°C in both primary and secondary analyses and a crystallizing temperature at 65°C in the secondary analysis. The compounds of Example 5 were identified to stably exist as amorphous compared with the conventional material. Therefore, the organic light emitting diodes including the compound of the present invention turned out to reduce influence of Joule heat generated during the operation, and thereby can have an improved life-span characteristic compared with a conventional organic light emitting diode.

## Claims

1. A compound for an organic photoelectric device, represented by the following Chemical Formula 112: 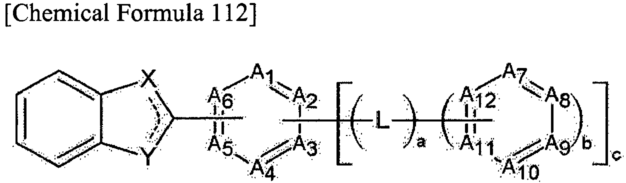 wherein, in Chemical Formula 112,
A₁ to A₁₂ are the same or different, and independently selected from the group consisting of CR₁ to CR₁₂ and N, provided that at least one of A₇ to A₁₂ is N, and R₁ to R₆ adjacent to each other optionally forms a fused ring, and R₇ to R₁₂ adjacent to each other optionally forms a fused ring,
X is selected from the group consisting of O, S, Se and NR₁₃, Y is CR₁₄ or N, provided that when X is selected from the group consisting of O, S, and Se, than Y is N,
L is a substituted or unsubstituted C6 to C50 arylene,
a is 0 or 1, provided that when a is 0, A₁ to A₆ are each independently CR₁ to CR₆, and when a is 1, at least one of A₁ to A₆ is N,
b and c are the same or different, and independently an integer ranging from 1 to 3, and
R₁ to R₁₄ are the same or different, and independently selected from the group consisting of hydrogen, a halogen, a nitrile, a cyano, a nitro, an amide, a carbonyl, an ester, a substituted or unsubstituted alkyl, a substituted or unsubstituted alkylene, a substituted or unsubstituted alkoxy, a substituted or unsubstituted alkenyl, a substituted or unsubstituted alkenylene, a substituted or unsubstituted alkynyl, a substituted or unsubstituted alkynylene, a substituted or unsubstituted cycloalkyl, a substituted or unsubstituted cycloalkylene, a substituted or unsubstituted aryl, a substituted or unsubstituted arylene, a substituted or unsubstituted arylamine, a substituted or unsubstituted heteroarylamine, a substituted or unsubstituted heterocycle, a substituted or unsubstituted amino, BRR', or SiRR'R", wehrein R, R' and R"are the same or different, and each independently a substituted or unsubstituted alkyl, or a substituted or unsubstituted aryl.

2. A compound for an organic photoelectric device, represented by the following Chemical Formula 113: 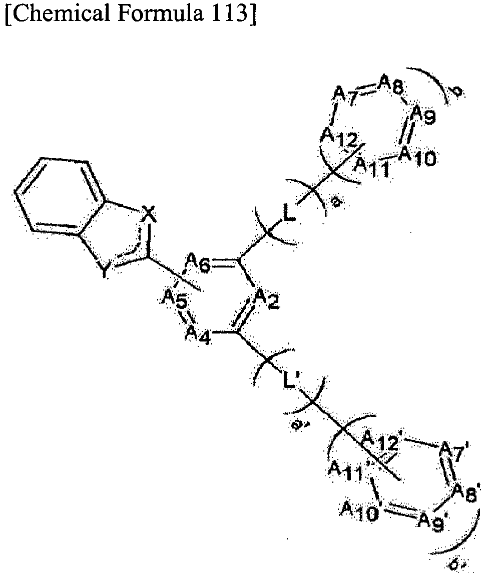 wherein, in Chemical Formula 113,
A₂, A₄ to A₁₂ and A₇' to A₁₂' are the same or different, and are each independently selected from the group consisting of CR₂, CR₄ to CR₁₂, CR₇' to CR₁₂' and N, provided at least one of A₇ to A₁₂ is N, and at least one of A₇' to A₁₂' is N, wherein R₄ to R₆ adjacent to each other optionally forms a fused ring, R₇ to R₁₂ adjacent to each other optionally forms a fused ring, and R₇' to R₁₂' adjacent to each other optionally forms a fused ring,
X is selected from the group consisting of O, S, Se and NR₁₃, Y is CR₁₄ or N, provided that when X is selected from the group consisting of O, S, and Se, and Y is N,
L and L' are the same or different, and are each independently is a substituted or unsubstituted C6 to C50 arylene,
a and a' are the same or different, and are each independently 0 or 1, provided that when a and a' are each independently 0, A₂, A₄ to A₆ are each independently CR₂, and CR₄ to CR₆, and when a and a' are each independently 1, at least one of A₂, and A₄ to A₆ is N,
b and b' are the same or different, and are each independently an integer ranging from 1 to 3, and
R₂, R₄ to R_{14,} and R₇' to R₁₂' are the same or different, and independently hydrogen, a halogen, a nitrile, a cyano, a nitro, an amide, a carbonyl, an ester, a substituted or unsubstituted alkyl, a substituted or unsubstituted alkylene, a substituted or unsubstituted alkoxy, a substituted or unsubstituted alkenyl, a substituted or unsubstituted alkenylene, a substituted or unsubstituted alkynyl, a substituted or unsubstituted alkynylene, a substituted or unsubstituted cycloalkyl, a substituted or unsubstituted cycloalkylene, a substituted or unsubstituted aryl, a substituted or unsubstituted arylene, a substituted or unsubstituted arylamine, a substituted or unsubstituted hetero arylamine, a substituted or unsubstituted heterocycle, a substituted or unsubstituted amino, BRR', or SiRR'R", wehrein R, R' and R"are the same or different, and each independently a substituted or unsubstituted alkyl, or a substituted or unsubstituted aryl.

3. The compound of claims 2, wherein the A₁ and A₅ are different from each other, and A₂ and A₄ are different from each other.

4. The compound of claim 1, wherein in Chemical Formula 112, R₁ to R₅, R' and R" are each independently a substituted or unsubstituted C6 to C40 aryl.

5. The compound of claims 1, wherein in Chemical Formula 1, R₁ to R₅, R' and R"are independently an arylamine selected from the group consisting of diphenyl amine, dinaphthyl amine, dibiphenyl amine, phenyl naphthyl amine, phenyl diphenyl amine, ditolyl amine, phenyl tolyl amine, carbazolyl, and triphenyl amine.

6. The compound of claims 1, wherein in Chemical Formula 1, R₁ to R₅, R' and R" are independently a heterocycle selected from the group consisting of a substituted or unsubstituted heterocycle, the heterocycle is selected from the group consisting of thiophenyl, furanyl, pyrrolyl, imidazolyl, thiazolyl, oxazolyl, oxadiazolyl, triazolyl, pyridinyl, pyridazinyl, quinolinyl, isoquinolinine, acridyl, imidazopyridinyl, and imidazopyrimidinyl.

7. The compound of claim 1, wherein in Chemical Formula 1, R₁ to R₅, R' and R"are independently, a substituted imidazolyl, or a substituted triazolyl, the substituent linked to nitrogen (N) of the imidazolyl or triazolyl is selected from the group consisting of a substituted or unsubstituted alkyl, a substituted or unsubstituted cycloalkyl, a substituted or unsubstituted aryl, and a substituted or unsubstituted heterocycle.

8. The compound of claim 1, wherein in Chemical Formula 112, one of R₇ to R₁₂ is substituted with a substituent selected from the group consisting of an amine-substituted alkyl, an amine-substituted cycloalkyl, an amine substituted aryl, and an amine-substituted heterocycle, and
in Chemical Formula 112, one of R₁ to R₆ is substituted with a substituent selected from the group consisting of a nitrile, a nitro, an amide, a carbonyl, and a substituted or unsubstituted heterocycle.

9. An organic photoelectric device, comprising
an anode, a cathode, and at least one organic thin layer between the anode and cathode,
wherein at least one of the organic thin layer comprises the compound according to one of claims 1 to 8.

10. The organic photoelectric device of claim 9, wherein the compound is a host material or a charge transport material.

11. The organic photoelectric device of claims 9 to 10, wherein at least one of the organic thin layer comprises the compound and a reducing dopant, which is selected from the group consisting of an alkaline metal, an alkaline earth metal, a rare earth element metal, an oxide of an alkaline metal, a halide of an alkaline metal, an organic complex of an alkaline metal, an oxide of an alkaline earth metal, a halide of an alkaline earth metal, an organic complex of an alkaline earth metal, an oxide of an alkaline earth metal, a halide of an alkaline earth metal, an organic complex of an alkaline earth metal, an oxide of a rare earth element metal, a halide of a rare earth element metal, and an organic complex of a rare earth element metal.

12. A display device comprising an organic photoelectric device according to claims 9 to 11.

## Patentansprüche

1. Verbindung für eine organische photoelektrische Vorrichtung der folgenden Chemischen Formel 112: 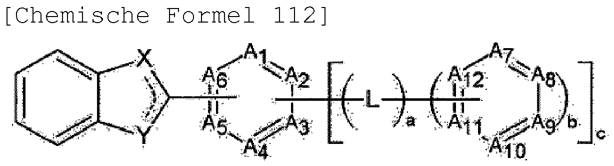 wobei in der Chemischen Formel 112:
A₁ bis A₁₂ gleich oder verschieden sind und unabhängig aus der Gruppe bestehend aus CR₁ bis CR₁₂ und N ausgewählt sind, mit der Maßgabe, dass mindestens eine der Variablen A₇ bis A₁₂ für N steht, und einander benachbarte Reste R₁ bis R₆ gegebenenfalls einen anellierten Ring bilden und einander benachbarte Reste R₇ bis R₁₂ gegebenenfalls einen anellierten Ring bilden,
X aus der Gruppe bestehend aus O, S, Se und NR₁₃ ausgewählt ist, Y für CR₁₄ oder N steht, mit der Maßgabe, dass dann, wenn X aus der Gruppe bestehend aus O, S und Se ausgewählt ist, Y für N steht,
L für ein substituiertes oder unsubstituiertes C6-bis C50-Arylen steht,
a für 0 oder 1 steht, mit der Maßgabe, dass dann, wenn a für 0 steht, A₁ bis A₆ jeweils unabhängig für CR₁ bis CR₆ stehen und dann, wenn a für 1 steht, mindestens eine der Variablen A₁ bis A₆ für N steht,
b und c gleich oder verschieden sind und unabhängig für eine ganze Zahl im Bereich von 1 bis 3 stehen und
R₁ bis R₁₄ gleich oder verschieden sind und unabhängig aus der Gruppe bestehend aus Wasserstoff, einem Halogen, einem Nitril, einem Cyano, einem Nitro einem Amid, einem Carbonyl, einem Ester, einem substituierten oder unsubstituierten Alkyl, einem substituierten oder unsubstituierten Alkylen, einem substituierten oder unsubstituierten Alkoxy, einem substituierten oder unsubstituierten Alkenyl, einem substituierten oder unsubstituierten Alkenylen, einem substituierten oder unsubstituierten Alkinyl, einem substituierten oder unsubstituierten Alkinylen, einem substituierten oder unsubstituierten Cycloalkyl, einem substituierten oder unsubstituierten Cycloalkylen, einem substituierten oder unsubstituierten Aryl, einem substituierten oder unsubstituierten Arylen, einem substituierten oder unsubstituierten Arylamin, einem substituierten oder unsubstituierten Heteroarylamin, einem substituierten oder unsubstituierten Heterocyclus, einem substituierten oder unsubstituierten Amino, BRR' oder SiRR'R", worin R, R' und R" gleich oder verschieden sind und jeweils unabhängig für ein substituiertes oder unsubstituiertes Alkyl oder ein substituiertes oder unsubstituiertes Aryl stehen, ausgewählt sind.

2. Verbindung für eine organische photoelektrische Vorrichtung der folgenden Chemischen Formel 113: 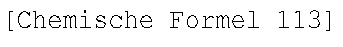 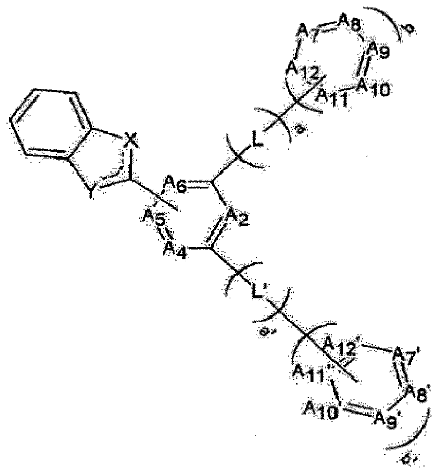 wobei in der Chemischen Formel 113:
A₂, A₄ bis A₁₂ und A₇' bis A₁₂' gleich oder verschieden sind und jeweils unabhängig aus der Gruppe bestehend aus CR₂, CR₄ bis CR₁₂, CR₇' bis CR₁₂' und N ausgewählt sind, mit der Maßgabe, dass mindestens eine der Variablen A₇ bis A₁₂ für N steht und mindestens eine der Variablen A₇' bis A₁₂' für N steht, wobei einander benachbarte Reste R₄ bis R₆ gegebenenfalls einen anellierten Ring bilden, einander benachbarte Reste R₇ bis R₁₂ gegebenenfalls einen anellierten Ring bilden und einander benachbarte Reste R₇' bis R₁₂' gegebenenfalls einen anellierten Ring bilden,
X aus der Gruppe bestehend aus O, S, Se und NR₁₃ ausgewählt ist, Y für CR₁₄ oder N steht, mit der Maßgabe, dass dann, wenn X aus der Gruppe bestehend aus O, S und Se ausgewählt ist, und Y N für steht,
L und L'gleich oder verschieden sind und jeweils unabhängig für ein substituiertes oder unsubstituiertes C6- bis C50-Arylen stehen,
a und a' gleich oder verschieden sind und jeweils unabhängig für 0 oder 1 stehen, mit der Maßgabe, dass dann, wenn a und a' jeweils unabhängig für 0 stehen, A₂, A₄ bis A₆ jeweils unabhängig für CR₂ und CR₄ bis CR₆ stehen und dann, wenn a und a' jeweils unabhängig für 1 stehen, mindestens eine der Variablen A₂ und A₄ bis A₆ für N steht,
b und b' gleich oder verschieden sind und jeweils unabhängig für eine ganze Zahl im Bereich von 1 bis 3 stehen und
R₂, R₄ bis R₁₄ und R₇' bis R₁₂' gleich oder verschieden sind und unabhängig für Wasserstoff, ein Halogen, ein Nitril, ein Cyano, ein Nitro ein Amid, ein Carbonyl, einen Ester, ein substituiertes oder unsubstituiertes Alkyl, ein substituiertes oder unsubstituiertes Alkylen, ein substituiertes oder unsubstituiertes Alkoxy, ein substituiertes oder unsubstituiertes Alkenyl, ein substituiertes oder unsubstituiertes Alkenylen, ein substituiertes oder unsubstituiertes Alkinyl, ein substituiertes oder unsubstituiertes Alkinylen, ein substituiertes oder unsubstituiertes Cycloalkyl, ein substituiertes oder unsubstituiertes Cycloalkylen, ein substituiertes oder unsubstituiertes Aryl, ein substituiertens oder unsubstituiertes Arylen, ein substituiertes oder unsubstituiertes Arylamin, ein substituiertes oder unsubstituiertes Heteroarylamin, ein substituierten oder unsubstituierten Heterocyclus, ein substituiertes oder unsubstituiertes Amino, BRR' oder SiRR'R", worin R, R' und R" gleich oder verschieden sind und jeweils unabhängig für ein substituiertes oder unsubstituiertes Alkyl oder ein substituiertes oder unsubstituiertes Aryl stehen, stehen.

3. Verbindung nach Anspruch 2, wobei A₁ und A₅ voneinander verschieden sind und A₂ und A₄ voneinander verschieden sind.

4. Verbindung nach Anspruch 1, wobei in der Chemischen Formel 112 R₁ bis R₅, R' und R" jeweils unabhängig für ein substituiertes oder unsubstituiertes C6- bis C40-Aryl stehen.

5. Verbindung nach Anspruch 1, wobei in der Chemischen Formel 1 R₁ bis R₅, R' und R" unabhängig für ein Arylamin aus der Gruppe bestehend aus Diphenylamin, Dinaphthylamin, Dibiphenylamin, Phenylnaphthylamin, Phenyl-diphenylamin, Ditolylamin, Phenyltolylamin, Carbazolyl und Triphenylamin stehen.

6. Verbindung nach Anspruch 1, wobei in der Chemischen Formel 1 R₁ bis R₅, R' und R" unabhängig für einen Heterocyclus aus der Gruppe bestehend aus einem substituierten oder unsubstituierten Heterocyclus stehen und der Heterocyclus aus der Gruppe bestehend aus Thiophenyl, Furanyl, Pyrrolyl, Imidazolyl, Thiazolyl, Oxazolyl, Oxadiazolyl, Triazolyl, Pyridinyl, Pyridazinyl, Chinolinyl, Isochinolinin, Acridyl, Imidazopyridinyl und Imidazopyrimidinyl ausgewählt ist.

7. Verbindung nach Anspruch 1, wobei in der Chemischen Formel 1 R₁ bis R₅, R' und R" unabhängig für ein substituiertes Imidazolyl oder ein substituiertes Triazolyl stehen und der an Stickstoff (N) des Imidazolyls bzw. Triazolyls gebundene Substituent aus der Gruppe bestehend aus einem substituierten oder unsubstituierten Alkyl, einem substituierten oder unsubstituierten Cycloalkyl, einem substituierten oder unsubstituierten Aryl und einem substituierten oder unsubstituierten Heterocyclus ausgewählt ist.

8. Verbindung nach Anspruch 1, wobei in der Chemischen Formel 112 einer der Reste R₇ bis R₁₂ durch einen Substituenten aus der Gruppe bestehend aus einem aminsubstituierten Alkyl, einem aminsubstituierten Cycloalkyl, einem aminsubstituierten Aryl und einem aminsubstituierten Heterocyclus substituiert ist und
in der Chemischen Formel 112 einer der Reste R₁ bis R₆ durch einen Substituenten aus der Gruppe bestehend aus einem Nitril, einem Nitro, einem Amid, einem Carbonyl und einem substituierten oder unsubstituierten Heterocyclus substituiert ist.

9. Organische photoelektrische Vorrichtung, umfassend
eine Anode, eine Kathode und mindestens eine organische dünne Schicht zwischen der Anode und Kathode,
wobei mindestens eine der organischen dünnen Schichten die Verbindung nach einem der Ansprüche 1 bis 8 umfasst.

10. Organische photoelektrische Vorrichtung nach Anspruch 9, wobei es sich bei der Verbindung um ein Wirtsmaterial oder ein Ladungstransportmaterial handelt.

11. Organische photoelektrische Vorrichtung nach den Ansprüchen 9 bis 10, wobei mindestens eine der organischen dünnen Schichten die Verbindung und einen reduzierenden Dotierstoff, der aus der Gruppe bestehend aus einem Alkalimetall, einem Erdalkalimetall, einem Seltenerdelementmetall, einem Oxid eines Alkalimetalls, einem Halogenid eines Alkalimetalls, einem organischen Komplex eines Alkalimetalls, einem Oxid eines Erdalkalimetalls, einem Halogenid eines Erdalkalimetalls, einem organischen Komplex eines Erdalkalimetalls, einem Oxid eines Erdalkalimetalls, einem Halogenid eines Erdalkalimetalls, einem organischen Komplex eines Erdalkalimetalls, einem Oxid eines Seltenerdelementmetalls, einem Halogenid eines Seltenerdelementmetalls und einem organischen Komplex eines Seltenerdelementmetalls ausgewählt ist, umfasst.

12. Anzeigevorrichtung, umfassend eine organische photoelektrische Vorrichtung nach den Ansprüchen 9 bis 11.

## Revendications

1. Composé pour un dispositif photoélectrique organique, représenté par la formule chimique 112 suivante : 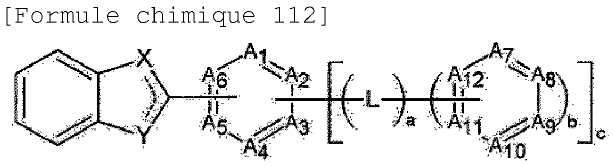 dans lequel, dans la formule chimique 112,
A₁ à A₁₂ sont identiques ou différents, et indépendamment choisis dans le groupe constitué par CR₁ à CR₁₂ et N, à condition qu'au moins un de A₇ à A₁₂ soit N, et les R₁ à R₆ adjacents les uns aux autres forment éventuellement un noyau condensé, et les R₇ à R₁₂ adjacents les uns aux autres forment éventuellement un noyau condensé,
X est choisi dans le groupe constitué par O, S, Se et NR₁₃, Y est CR₁₄ ou N, à condition que lorsque X est choisi dans le groupe constitué par O, S et Se, alors Y soit N,
L est un arylène en C6 à C50 substitué ou non substitué,
a vaut 0 ou 1, à condition que lorsque a vaut 0, A₁ à A₆ soient chacun indépendamment CR₁ à CR₆, et lorsque a vaut 1, au moins un de A₁ à A₆ soit N,
b et c sont identiques ou différents, et indépendamment un entier allant de 1 à 3, et
R₁ à R₁₄ sont identiques ou différents, et indépendamment choisis dans le groupe constitué par l'hydrogène, un halogène, un nitrile, un cyano, un nitro, un amide, un carbonyle, un ester, un alkyle substitué ou non substitué, un alkylène substitué ou non substitué, un alcoxy substitué ou non substitué, un alcényle substitué ou non substitué, un alcénylène substitué ou non substitué, un alcynyle substitué ou non substitué, un alcynylène substitué ou non substitué, un cycloalkyle substitué ou non substitué, un cycloalkylène substitué ou non substitué, un aryle substitué ou non substitué, un arylène substitué ou non substitué, une arylamine substituée ou non substituée, une hétéroarylamine substituée ou non substituée, un hétérocycle substitué ou non substitué, un amino substitué ou non substitué, BRR', ou SiRR'R", dans lesquels R, R' et R" sont identiques ou différents, et chacun indépendamment un alkyle substitué ou non substitué, ou un aryle substitué ou non substitué.

2. Composé pour un dispositif photoélectrique organique, représenté par la formule chimique 113 suivante : 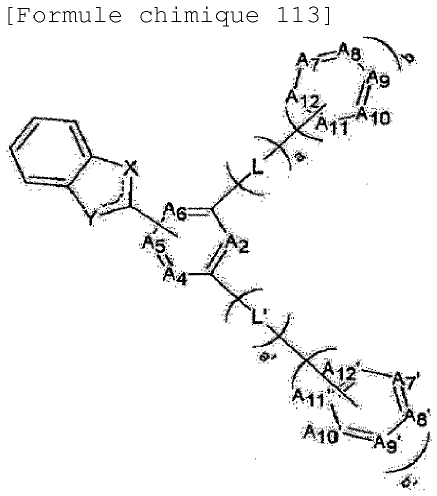 dans lequel, dans la formule chimique 113,
A₂, A₄ à A₁₂ et A'₇ à A'₁₂ sont identiques ou différents, et sont chacun indépendamment choisis dans le groupe constitué par CR₂, CR₄ à CR₁₂, CR'₇ à CR'₁₂ et N, à condition qu'au moins un de A₇ à A₁₂ soit N, et au moins un de A'₇ à A'₁₂ soit N, les R₄ à R₆ adjacents les uns aux autres formant éventuellement un noyau condensé, les R₇ à R₁₂ adjacents les uns aux autres forment éventuellement un noyau condensé, et les R'₇ à R'₁₂ adjacents les uns aux autres forment éventuellement un noyau condensé,
X est choisi dans le groupe constitué par O, S, Se et NR₁₃, Y est CR₁₄ ou N, à condition que lorsque X est choisi dans le groupe constitué par O, S et Se, et Y soit N,
L et L' sont identiques ou différents, et sont chacun indépendamment un arylène en C6 à C50 substitué ou non substitué,
a et a' sont identiques ou différents, et valent chacun indépendamment 0 ou 1, à condition que lorsque a et a' valent chacun indépendamment 0, A₂ et A₄ à A₆ soient chacun indépendamment CR₂ et CR₄ à CR₆, et lorsque a et a' valent chacun indépendamment 1, au moins un de A₂ et A₄ à A₆ soit N,
b et b' sont identiques ou différents, et sont chacun indépendamment un entier allant de 1 à 3, et
R₂, R₄ à R₁₄ et R'₇ à R'₁₂ sont identiques ou différents, et indépendamment l'hydrogène, un halogène, un nitrile, un cyano, un nitro, un amide, un carbonyle, un ester, un alkyle substitué ou non substitué, un alkylène substitué ou non substitué, un alcoxy substitué ou non substitué, un alcényle substitué ou non substitué, un alcénylène substitué ou non substitué, un alcynyle substitué ou non substitué, un alcynylène substitué ou non substitué, un cycloalkyle substitué ou non substitué, un cycloalkylène substitué ou non substitué, un aryle substitué ou non substitué, un arylène substitué ou non substitué, une arylamine substituée ou non substituée, une hétéroarylamine substituée ou non substituée, un hétérocycle substitué ou non substitué, un amino substitué ou non substitué, BRR', ou SiRR'R", dans lesquels R, R' et R" sont identiques ou différents, et chacun indépendamment un alkyle substitué ou non substitué, ou un aryle substitué ou non substitué.

3. Composé selon la revendication 2, dans lequel les A₁ et A₅ sont différents l'un de l'autre, et A₂ et A₄ sont différents l'un de l'autre.

4. Composé selon la revendication 1 dans lequel, dans la formule chimique 112, R₁ à R₅, R' et R" sont chacun indépendamment un aryle en C6 à C40 substitué ou non substitué.

5. Composé selon la revendication 1 dans lequel, dans la formule chimique 1, R₁ à R₅, R' et R" sont indépendamment une arylamine choisie dans le groupe constitué par la diphénylamine, la dinaphtylamine, la dibiphénylamine, la phénylnaphtylamine, la phényl-diphénylamine, la ditolylamine, la phényltolylamine, le carbazolyle, et la triphénylamine.

6. Composé selon la revendication 1 dans lequel, dans la formule chimique 1, R₁ à R₅, R' et R" sont indépendamment un hétérocycle choisi dans le groupe constitué par un hétérocycle substitué ou non substitué, l'hétérocycle est choisi dans le groupe constitué par le thiophényle, le furanyle, le pyrrolyle, l'imidazolyle, le thiazolyle, l'oxazolyle, l'oxadiazolyle, le triazolyle, le pyridinyle, le pyridazinyle, le quinolinyle, l'isoquinolinine, l'acridyle, l'imidazopyridinyle, et l'imidazopyrimidinyle.

7. Composé selon la revendication 1 dans lequel, dans la formule chimique 1, R₁ à R₅, R' et R" sont indépendamment un imidazolyle substitué ou un triazolyle substitué, le substituant lié à l'azote (N) de l'imidazolyle ou du triazolyle est choisi dans le groupe constitué par un alkyle substitué ou non substitué, un cycloalkyle substitué ou non substitué, un aryle substitué ou non substitué, et un hétérocycle substitué ou non substitué.

8. Composé selon la revendication 1 dans lequel, dans la formule chimique 112, un de R₇ à R₁₂ est substitué par un substituant choisi dans le groupe constitué par un alkyle substitué par une amine, un cycloalkyle substitué par une amine, un aryle substitué par une amine, et un hétérocycle substitué par une amine, et
dans la formule chimique 112, un de R₁ à R₆ est substitué par un substituant choisi dans le groupe constitué par un nitrile, un nitro, un amide, un carbonyle, et un hétérocycle substitué ou non substitué.

9. Dispositif photoélectrique organique, comprenant
une anode, une cathode, et au moins une couche mince organique entre l'anode et la cathode,
dans lequel au moins une des couches minces organiques comprend le composé selon l'une des revendications 1 à 8.

10. Dispositif photoélectrique organique selon la revendication 9, dans lequel le composé est un matériau hôte ou un matériau de transport de charges.

11. Dispositif photoélectrique organique selon les revendications 9 à 10, dans lequel au moins une des couches minces organiques comprend le composé et un dopant réducteur, qui est choisi dans le groupe constitué par un métal alcalin, un métal alcalino-terreux, un métal des terres rares, un oxyde d'un métal alcalin, un halogénure d'un métal alcalin, un complexe organique d'un métal alcalin, un oxyde d'un métal alcalino-terreux, un halogénure d'un métal alcalino-terreux, un complexe organique d'un métal alcalino-terreux, un oxyde d'un métal alcalino-terreux, un halogénure d'un métal alcalino-terreux, un complexe organique d'un métal alcalino-terreux, un oxyde d'un métal des terres rares, un halogénure d'un métal des terres rares, et un complexe organique d'un métal des terres rares.

12. Dispositif d'affichage comprenant le dispositif photoélectrique organique selon les revendications 9 à 11.
